# EUROPÄISCHE PATENTANMELDUNG

(11) **EP 1 734 113 A1**
(43) Veröffentlichungstag der Anmeldung: **20.12.2006**
(21) Anmeldenummer: 06115206.2
(22) Anmeldetag: 09.06.2006
(51) Int. Cl.: C12N 5/06, A61L 27/60, A61K 35/36

(54) **Verfahren zur Erzeugung eines Hautäquivalents**

(30) Priorität: 17.06.2005 DE 102005028899
(71) Anmelder: GKSS-Forschungszentrum Geesthacht GmbH, 21502 Geesthacht (DE)
(72) Erfinder: Boese, Gregor, Dr., 12047 Berlin (DE); Wilke, Andreas, Dr., 14197 Berlin (DE); Richau, Klaus, Dr., 12435 Berlin (DE); Albrecht, Wolfgang, Dr., 14513 Teltow (DE); Lendlein, Andreas, Prof. Dr., 14167 Berlin (DE)
(74) Vertreter: Reinstädler, Diane

(57) **Zusammenfassung**

Die Erfindung betrifft ein Verfahren zur Erzeugung einer organotypischen Hybridstruktur (34) der Haut. Erfindungsgemäß wird zumindest ein Zelltyp (10) auf einer Hohlfasermembran (20) immobilisiert und anschließend in dynamischer Kultur über eine erste Kultivierungsdauer kultiviert, so dass eine dreidimensionale organotypische Hybridstruktur (34) der Haut gebildet wird. Die Erfindung betrifft ferner eine nach dem Verfahren hergestellte Hybridstruktur (34), insbesondere eine epidermale Struktur (36) und/oder dermale Hautstruktur (38) auf einer Polymerhohlfaser (22), sowie die Verwendung der Hybridstruktur (34) für den Hautersatz oder in-vitro Untersuchungen, insbesondere für in-vitro Toxizitätstests.

## Beschreibung

Die Erfindung betrifft ein Verfahren zur Erzeugung einer organotypischen Hybridstruktur der Haut. Erfindungsgemäß wird zumindest ein Zelltyp auf einer Hohlfasermembran immobilisiert und anschließend in einer dynamischen Kultur über eine erste Kultivierungsdauer kultiviert, so dass eine dreidimensionale organotypische Hybridstruktur der Haut gebildet wird. Die Erfindung betrifft ferner eine nach dem Verfahren hergestellte Hybridstruktur, insbesondere eine epidermale Struktur und/oder dermale Hautstruktur auf einer Polymerhohlfaser, sowie die Verwendung der Hybridstruktur für den Hautersatz oder in-vitro Untersuchungen, insbesondere für in-vitro Toxizitätstests.

Auf dem Gebiet des Tissue Engineering, das heißt künstlich kultivierten Gewebestrukturen, wird seit langem nach Lösungen zum Aufbau körpereigener Gewebe gesucht. Wichtige Anwendungsbereiche in-vitro kultivierter Gewebesubstitute stellen ihre Verwendung für die Transplantation oder für in-vitro Untersuchungen dar. Beispielsweise erfordern sicherheitstoxikologische Prüfungen neuer Stoffe oder Inhaltsstoffe für die Gewährleistung von Arbeitsschutz und Verbraucherschutz sowie Prüfungen neuer Stoffe auf Umweltverträglichkeit geeignete Testssysteme. Für Verträglichkeitsprüfungen von kosmetischen Inhaltsstoffen sind z. B. sechs sicherheitstoxikologische Prüfungsmethoden vorgeschrieben, zu denen die akute Toxizität (dermal und oral), die Augenreizung bzw. -ätzung, die Hautreizung bzw. -ätzung, die Sensibilisierung der Haut, die Hautpenetration und die Genotoxizität/Mutagenität zählen. Sensibilisierende und allergische Wirkungen sind dabei die häufigste Eigenschaft chemischer Stoffe, die bei ca. 10 % auftreten. Bis heute werden für solche toxikologischen Analysen vielfach Tierversuche durchgeführt, indem z. B. Substanzen unter die Haut von Meerschweinchen injiziert und anschließend immunologische Reaktionen an der geschädigten Haut analysiert werden. Als Alternative zu Tierversuchen oder teuren klinischen Studien können in durch Tissue Engineering erzeugten organotypischen Testmodellen Wundheilung, Alterung oder Hautirritationen, die durch physikalische oder chemische Einflüsse verursacht sind, untersucht werden.

Tierversuchsfreie Prüfungen von Inhaltsstoffen konnten beispielsweise auf Penetration durch die Haut, auf ätzende und phototoxische Eigenschaften sowie Mutagenität etabliert werden.

Es ist bekannt, dass Monolayer-Zellkulturen für zahlreiche in-vitro Prüfungen geeignet sind, deren Aussagekraft jedoch nicht zur Analyse komplexer physiologischer Prozesse ausreicht.

Es ist auch ein dreidimensionales Hautmodell unter der Handelsbezeichnung EpiSkin beschrieben, bei dem es sich um ein in-vitro rekonstruiertes humanes Epithelium handelt, das ein funktionsfähiges Stratum corneum enthält. Nachteilig ist jedoch, dass EpiSkin im statischen Milieu einer Kulturschale gezüchtet wird, die eine kontrollierte Versorgung der Gewebekultur unmöglich macht.

Ein weiteres Hautmodell ist unter der Handelsbezeichnung EpiDerm bekannt, das auf humanen epidermalen Keratinozyten basiert. Es besteht aus 8 bis 12 Zellschichten, die das Stratum basale, Stratum spinosum und Stratum granulosum bilden, und 10 bis 15 Schichten des Stratum corneum. Weitere epidermale Zellen, Blutzellen und Hautschichten sind nicht vorhanden, so dass nur eine eingeschränkte Testsensibilisierung, keine Regenerationsfähigkeit und eine Lebensspanne von maximal drei Wochen erzielt werden. Die Zellen werden an der Gas-/Flüssigkeitsgrenzfläche einer statischen Kultur mit den beschriebenen Nachteilen einer schlechten Nähr- und Sauerstoffversorgung kultiviert.

Während folglich die Kultivierung der Epidermis im statischen Milieu über eine begrenzte Anzahl an Schichten gelingt, ist bisher kein befriedigender Ersatz der darunter liegenden Dermis verfügbar. Prüfungen auf hautreizende und hautsensibilisierende Eigenschaften sind bis heute ohne Tierexperimente nicht möglich.

Des Weiteren sind in der Literatur hybride Hautersatzprodukte offenbart (US 5,460,939 A; US 5,489,304 A), die eine biologisch abgeleitete Komponente und ein synthetisches, stabiles Material enthalten. Hier ist festzustellen, dass die derzeit verfügbaren Produkte entweder spezielle Vorrichtungen, Träger oder die Zugabe von wachstumsfördernden Verbindungen benötigen, die Fremdstoffe für den Körper darstellen, oder viele Verfahrensschritte beinhalten und damit verhältnismäßig teuer in der Herstellung sind.

Es ist ebenfalls bekannt, artifizielles Gewebe unter dynamischen Kulturbedingungen zu produzieren, um die Zellschichten gleichmäßig mit Kulturmedium zu versorgen. Die Kultivierung kann kontinuierlich oder in definierten Impulsen gestaltet werden, wodurch die Bildung von ungerührten Schichten zwischen Zellen und Biomaterialien minimiert wird. In Weiterentwicklung von Flachbettbioreaktoren können die mit Zellen besiedelten Biomaterialien z. B. in einen Träger eingespannt werden und im Zentrum eines Perfusionsbioreaktors reifen. Bei einer anderen Möglichkeit kann Gewebe an der Grenze zu Hohlfasern in einem Perfusionsbioreaktor herangezogen werden. Diese Hohlfaserreaktoren sind speziell an die zu kultivierenden Zellen angepasst, so dass es sich jeweils um Einzellösungen handelt. Die Optimierung eines Hohlfaserreaktors für die spezifische Anwendung ist technisch sehr aufwendig.

Es ist beispielsweise bekannt, dass Epithelgewebe in Gradientenkultur gezüchtet werden kann. DE 199 43 205 C1 offenbart hier ein Verfahren zur Verstoffwechslung und zum Transport von Stoffen, in dem polar orientierte Zellen, wie z. B. Nierenepithelzellen oder Leberzellen, im Raum zwischen zwei koaxial ineinander angeordneten Hohlfasermembranen kultiviert werden, so dass ein Stoffgradient aufgebaut wird und ein gerichteter Transport stattfindet. Die Zellen wachsen in Polyschichten, ohne jedoch eine höhere Gewebestruktur zu generieren.

Durch Unger et al. (2005) Biomaterials 26, 1877-1884, ist auch bekannt, dass humane Zelllinien verschiedener Gewebe und Zelltypen auf Hohlfasern aus Polyethersulfon (PES) wachsen und sich verbreiten. Jedoch stoppt das Wachstum mit Erreichen einer konfluenten Monoschicht.

Unger et al. (2005) Biomaterials 26, 3461-3469, konnten des Weiteren ein adhärentes Wachstum von Endothelzellen auf beschichteten PES-Hohlfasern zeigen. Für die Vaskularisation von Transplantaten wird das Animpfen von Biomaterial mit Endothelzellen bzw. eine Co-Kultivierung vorgeschlagen, ohne hierfür eine konkrete Lösung zu beschreiben. Auch werden PES-Bioreaktoren für die quantitative Herstellung von spezifischen Zelltypen oder Stammzellen empfohlen, womit jedoch nicht die Intention der Generierung höherer Gewebestrukturen verfolgt wird.

Aufgabe der vorliegenden Erfindung war es, ein Verfahren zur Erzeugung einer organotypischen Hybridstruktur der Haut bereitzustellen, mit dem standardisiertes, dreidimensionales Gewebe einfach und kostengünstig hergestellt werden kann. Insbesondere soll eine gezielte, kontrollierte und gleichmäßige Gewebereifung erreicht werden. Die so hergestellte Hybridstruktur soll für in-vitro Toxizitätstests und die Analyse komplexer physiologischer Prozesse, insbesondere im Zusammenhang mit einer Vaskularisation, dienen.

Die Aufgabe der Erfindung wird gemäß den unabhängigen Ansprüchen gelöst. Die Unteransprüche beinhalten bevorzugte Ausführungsformen.

Erfindungsgemäß wird ein Verfahren zur Erzeugung einer organotypischen Hybridstruktur der Haut bereitgestellt, das folgende Schritte umfasst:
(a) Immobilisierung von zumindest einem ersten Zelltyp auf einer Hohlfasermembran, und
(b) Kultivierung des zumindest einen ersten Zelltyps in dynamischer Kultur über eine erste Kultivierungsdauer zu einer mehrschichtigen organotypischen Gewebestruktur der Haut.

Überraschenderweise wurde gefunden, dass Hautzellen, die auf einer Hohlfasermembran immobilisiert werden, auch zu höheren Gewebestrukturen reifen können. Indem die Hohlfasermembran kontinuierlich mit Medium um-/durchströmt wird, ist eine optimale Versorgung aller Zellen in Polyschichten mit Nährstoffen realisierbar. Insbesondere die durch die Hohlfasermembran hervorgerufene Kompartimentierung des Bioreaktors ermöglicht eine optimale Mediendosierung durch die Hohlfaser, während eine gezielte Sauerstoffexposition im äußeren Kompartiment Keratinozyten zur Stratifizierung anregt. Weiterhin können verschiedene Zelltypen in unterschiedlichen Bereichen der Hohlfasermembran gezielt co-kultiviert werden und in deren Gesamtheit zu einer dreidimensionalen Hybridstruktur heranwachsen. Die Reifung der Hybridstruktur und die Aufrechterhaltung der Vitalität sind in wenigen Verfahrensschritten und in einem einzelnen Bioreaktor einfach zu bewerkstelligen. Die dynamische Kultur gewährleistet nicht nur eine lange Lebensdauer der Hybridstruktur, wobei durch die Verwendung von Stammzellen die Regenerationsfähigkeit des Gewebes noch einmal verbessert werden kann, sondern auch die reproduzierbare Erzeugung standardisierter Hybridstrukturen. Wenn das Medium mit verschiedenen Wirksubstanzen versetzt wird, können vorteilhaft im selben Bioreaktor zellspezifische Reaktionen, wie z. B. Differenzierung und Angiogenese, eingeleitet, dermatologische Untersuchungen, wie z. B. standardisierte Toxizitätstests, durchgeführt und physiologische Prozesse analysiert werden.

Im Sinne der Erfindung bedeutet "Hybridstruktur" eine Assoziation von biologischem Material und synthetischen Stoffen. Unter biologischem Material werden insbesondere intakte lebensfähige Zellen verstanden, die isoliert und in Zellverbänden auftreten können, wobei die Zellverbände Agglomerate, Polyschichten oder höhere Strukturen, wie z. B. Gewebe, Organe, Organismen etc., darstellen können. Das synthetische Material kann organischen oder anorganischen Ursprungs, chemisch oder biologisch hergestellt, und resorbierbar oder nicht resorbierbar sein.

"Zelltyp" bezeichnet vorliegend eine Zelle, die durch ihren Genotyp, wie z. B. Karyotyp, und Phänotyp, wie z. B. Proliferation, Differenzierung, Stoffwechsel und Morphologie, charakterisiert ist. Es kann sich hierbei um intakte Organismen, wie z. B. Mikroorganismen, handeln, deren Arten sich untereinander sowohl im Genotyp als auch Phänotyp unterscheiden, oder um Bestandteile eines Organismus, deren Zellen einen identischen Genotyp, aber einen unterschiedlichen Phänotyp aufweisen. Zu Letzteren zählen beispielsweise Stammzellen, Progenitorzellen und ausdifferenzierte Zellen, wie z. B. Endothelzellen, Hepatozyten, Keratinozyten, Fibroblasten etc. Der Zelltyp kann durch Biopsie von Primärzellen, Zellkultur oder als Zelllinie verfügbar sein.

"Dynamische Kultur" oder "Perfusionskultur" werden hierin austauschbar verwendet und beinhalten eine kontinuierliche Prozessführung, indem Medium permanent oder in periodischen Intervallen einem entsprechenden Perfusionsbioreaktor am Zulauf zugegeben und gleichzeitig am Ablauf aus dem Bioreaktor entfernt wird, wodurch ein im Bioreaktor befindlicher Gewebeträger so durchströmt wird, dass sich Zellen ansiedeln, wachsen, verbreiten und gegebenenfalls differenzieren können. Der Austausch des Kulturmediums betrifft dabei sowohl die Gasphase als auch die Flüssigphase.

Sofern keine Einzelzellsuspension vorliegt, wird eine Probe, wie z. B. eine Hautgewebeprobe, zunächst durch mechanische und/oder proteolytische Prozesse in eine Einzelzellsuspension überführt. Anschließend werden die Einzelzellen auf einer Hohlfasermembran immobilisiert. Dabei können entweder alle Zellen oder mindestens ein bestimmter Zelltyp fixiert werden, sofern im letzteren Fall entweder der zumindest eine erste Zelltyp oder die Hohlfasermembran so markiert sind, dass eine selektive Assoziation möglich ist. Um die nachfolgende Generierung einer Hybridstruktur der Haut zu gewährleisten, muss zumindest ein auf der Hohlfasermembran immobilisierter Zelltyp eine Stammzelle, Progenitorzelle der Haut oder ausdifferenzierte Hautzelle beinhalten. Diffusionsprozesse sind für den Stofftransport, vorliegend den Transport der Zellen in Lösung an die feste Oberfläche der Hohlfasermembran, verantwortlich. Für eine gleichmäßige Verteilung der Zellen bzw. ersten Zelltypen auf der Hohlfasermembran ist es vorteilhaft, den Prozess der Immobilisierung so durchzuführen, dass sich Zellen und Hohlfasermembran in einer Lösung befinden, indem beispielsweise die Hohlfasermembran in die Zellsuspension eingebracht wird, wie z. B. durch Eintauchen, Umspülen oder Fixierung im Bioreaktor. Die Lösung umfasst geeignete Komponenten, die die Überlebensfähigkeit der ersten Zelltypen sichern, wie z. B. C-Quelle, N-Quelle, Mineralien, Puffer etc. Vorzugsweise handelt es sich um ein Nährmedium oder eine physiologische Kochsalzlösung. Entsprechende Lösungen sind dem Fachmann ebenso bekannt wie geeignete Verfahren der Immobilisierung von Zellen. Insbesondere sei hier das adhärente Wachstum von Zellen genannt, wofür Serumzusätze oder die Beschichtung der Hohlfasermembran mit Adhäsionsfaktoren notwendig sind. In Abhängigkeit vom Einbringen der Zelltypen in verschiedene Kompartimente und der Porengröße der Hohlfasermembran können das Lumen, der Außenbereich und/oder die Poren besiedelt werden. Die Hohlfasermembran wiederum ist so ausgewählt, dass sie den Anforderungen der späteren Anwendung der generierten Gewebestruktur genügt. Während Hauttransplantate vorwiegend resorbierbare Materialien der Hohlfasermembran verwenden, sind für Hautsubstitute in dermatologischen und pharmakologischen Testreihen nicht resorbierbare Materialien bevorzugt.

Die immobilisierten Zellen werden anschließend mit einer geeigneten Nährlösung, die sich von der Immobilisierungslösung unterscheiden kann, in Perfusionskultur kultiviert. Die Dauer der ersten Kultivierung wird dabei von der gewünschten Zelldichte und -dicke bestimmt. Vorliegend können das Lumen und der Außenbereich der Hohlfasermembran sowohl einzeln als auch zusammen durchströmt werden. Darüber hinaus können für die verschiedenen Kompartimente ein und dasselbe, aber auch mehrere unterschiedliche Kulturmedien genutzt werden. Insbesondere Epithelzellen, wie sie die Keratinozyten der epidermalen Haut darstellen, kommen in-vivo an Stellen vor, an denen die eine Gewebeseite einem anderen Milieu ausgesetzt ist als die andere. Zur Simulierung dieser speziellen Gewebesituation in-vitro werden Hohlfasermembranen mit einer Porengröße unterhalb des mittleren Zelltypdurchmessers ausgewählt und das resultierende luminale sowie basale Kompartiment separat mit Nährlösung und gegebenenfalls wie unter natürlichen Bedingungen durchströmt. Zunächst wachsen die Zellen in einer Monoschicht, die durch nachfolgendes adhärentes Zellwachstum mittels Expression von Zelladhäsionsmolekülen, wie z. B. E-Selectin, ICAM-1 und/oder VCAM-1, konfluent wird. Der Übergang in Polyschichten vollzieht sich unter den speziellen Bedingungen der Bioreaktorkultur, wobei die Initiation durch Entfernen des Mediums im Außenbereich und Sauerstoffexposition der Keratinozyten vollzogen werden kann. Die erzielte Zelldichte respektive -dicke ist dabei vorzugsweise über die gesamte Hohlfasermembran konstant. Das resultierende Hautäquivalent aus mehreren, dreidimensionalen Zellschichten ist organotypisch.

Zur verbesserten Angleichung an sein natürliches Pendant werden bevorzugt mehrere erste Zelltypen co-immobilisiert, Co-Kulturen angezüchtet und/oder Stammzellen integriert, deren kontrollierte Differenzierung beliebige Gewebestrukturen und die Regeneration und anhaltende Vitalität der Hybridstruktur sicherstellt. Es versteht sich, dass sich mehrere erste Zelltypen im Genotyp und/oder Phänotyp untereinander unterscheiden.

Den erfindungsgemäßen Verfahrensschritten können sich nach Schritt (b) weitere Schritte anschließen:
(c) Immobilisierung von zumindest einem zweiten Zelltyp auf der Hybridstruktur und/oder auf der Hohlfasermembran,
(d) Kultivierung des zumindest einen zweiten Zelltyps in dynamischer Kultur über eine zweite Kultivierungsdauer, und optional
(e) Wiederholung der Schritte (c) und (d) mit zumindest einem weiteren Zelltyp.

Das Vorgehen der Schritte (c) und (d) korrespondiert weitestgehend mit dem der Schritte (a) und (b), wie es bereits im Verlauf dieser Spezifikation beschrieben wurde. Der zweite Zelltyp unterscheidet sich im Genotyp und/oder Phänotyp von den bereits immobilisierten und kultivierten ersten Zelltypen der Schritte (a) und (b), wobei hinsichtlich der Verwendung von Stammzellen deren auszudifferenzierender Phänotyp maßgeblich ist. Der zweite Zelltyp ist dabei keineswegs auf differenzierte Hautzellen oder zu solchen auszudifferenzierende Zellen beschränkt. Diese Unterscheidung muss auf zumindest einen zweiten Zelltyp zutreffen. Wie in Schritt (a) ist in Schritt (c) eine Co-Immobilisierung möglich. Der oder die anderen zu immobilisierenden zweiten Zelltypen können untereinander sämtlich in Genotyp und/oder Phänotyp verschiedene Zelltypen sein, aber auch einem oder mehreren ersten Zelltypen der vorangegangenen Schritte entsprechen, sofern zumindest ein zweiter Zelltyp vertreten ist. Die Co-Immobilisierungen sind insbesondere für die authentische Hauterzeugung erforderlich, da Epidermis und Dermis aus mehreren Zelltypen bestehen, deren Assoziation und Interaktion die Eigenschaften der jeweiligen Hautschicht bedingen. Von besonderer Bedeutung ist die Vaskularisation der Hautschichten, so dass sich eine Co-Immobilisierung und Co-Kultivierung mit Endothelzellen als einem ersten Zelltyp, einem zweiten Zelltyp, einzigem zweiten Zelltyp oder einem weiteren Zelltyp anbietet.

Der zumindest eine zweite Zelltyp kann sowohl adhärent auf der existierenden Hybridstruktur des ersten Zelltyps angesiedelt und angezüchtet werden als auch in einem bisher nicht besiedelten Kompartiment der Hohlfasermembran, das heißt entweder dem Lumen oder dem Außenbereich, vorzugsweise dem Lumen. Die selektive Besiedlung verschiedener Kompartimente bietet den Vorteil der separaten Dosierung verschiedener Medien, die optimal auf das Wachstum des jeweiligen Zelltyps abgestimmt sind. Vorzugsweise werden als Zelltyp in Schritt (a) Keratinozyten auf der außen liegenden Oberfläche der Hohlfasermembran und als zweiter Zelltyp in Schritt (c) Fibroblasten auf der innen liegenden Oberfläche der Hohlfasermembran immobilisiert und kultiviert. Alternativ können als erster Zelltyp in Schritt (a) Fibroblasten auf der außen liegenden Oberfläche der Hohlfasermembran und als zweiter Zelltyp in Schritt (c) Keratinozyten auf der entstandenen Hybridstruktur angesiedelt werden. In diesem Fall müssen Immobilisierungslösung und Nährmedium den spezifischen Erfordernissen der Co-Kultur des ersten Zelltyps und des zweiten Zelltyps entsprechen. Des Weiteren können natürlich auch Stammzellen oder Progenitorzellen zu den vorgenannten Zelltypen ausdifferenziert werden. Die zweite Kultivierungsdauer wird vom zweiten Zelltyp bestimmt und kann von der ersten Kultivierungsdauer abweichen.

Die Immobilisierung und Kultivierung des zumindest einen zweiten Zelltyps berücksichtigt den Schichtcharakter der Haut, die sich im Wesentlichen aus Epidermis, Dermis und Subcutis aufbaut. Die Verfahrensschritte können beliebig oft wiederholt werden, bis die gewünschte Struktur des Hautäquivalents erreicht ist bzw. solange selbiges einfach, stabil und lebensfähig kultiviert werden kann. Es versteht sich, dass bei einer Wiederholung gemäß Schritt (e) zumindest ein weiterer Zelltyp sich von allen bisher in der Hybridstruktur enthaltenen Zelltypen unterscheiden soll, das heißt dem erstem, zweiten, dritten etc. Zelltyp vorheriger Schritte oder jeweils mehreren Zelltypen davon. Sofern der Schritt (e) zur Anwendung kommt, werden zunächst die Schritte (c) und (d) einmal mit zumindest einem weiteren (das heißt einem dritten) Zelltyp wiederholt. Optional kann anschließend erneut Schritt (e) und damit eine erneute Wiederholung von Immobilisierung und Kultivierung mit noch einem weiteren (das heißt dem vierten) Zelltyp stattfinden. Die weiteren zweiten Kultivierungsdauern sind dabei zellspezifisch. Die einzelnen zweiten Kultivierungsdauern werden in der vorliegenden Erfindung mit erster zweiter Kultivierungsdauer, zweiter zweiter Kultivierungsdauer, dritter zweiter Kultivierungsdauer etc. bezeichnet und sind nicht mit der dritten oder vierten Kultivierungsdauer im Sinne der Erfindung, wie sie nachfolgend noch beschrieben werden, zu verwechseln. Bevorzugt ist die einmalige Anwendung von Schritt (e), in Anschluss dessen das Verfahren beendet oder anderweitig fortgeführt wird.

Zur Aufrechterhaltung der Vitalität der Hybridstruktur, die aus zumindest einem ersten Zelltyp, gegebenenfalls zumindest einem zweiten Zelltyp und gegebenenfalls zumindest einem weiteren Zelltyp an biologischem Material besteht, kann sich nach Schritt (b), (d) und/oder (e) ein weiterer Schritt anschließen:
(f) Kultivierung der Hybridstruktur in dynamischer Kultur über eine dritte Kultivierungsdauer.

Die dynamische Kultur bietet optimale Voraussetzungen für die gleichmäßige und kontrollierte Versorgung aller Hautzellschichten mit Nährstoffen und Sauerstoff, wodurch die Lebensdauer der Hybridstruktur deutlich verlängert wird. Ist der Hybridstruktur die inhärente Eigenschaft der Regeneration, wie z. B. durch die Integration von Stammzellen, gegeben, kann im Zuge der kontinuierlichen Prozessführung eine beliebig lange Kultivierung erreicht werden. Abgestorbene Zellen werden mit dem Kulturmedium ausgetragen und durch ausdifferenzierte Hautzellen ersetzt. Die Schichtdicke der Hybridstruktur bleibt hierbei konstant. Das Kulturmedium muss der spezifischen Hybridstruktur der Haut ebenso angepasst werden wie die Prozessbedingungen und die Auslegung des Bioreaktors. Geeignete Optimierungsstrategien hierzu sind dem Fachmann bekannt.

In einer Ausführungsform des vorliegenden Verfahrens werden als der zumindest eine erste Zelltyp, als der zumindest eine zweite Zelltyp und/oder als der zumindest eine weitere Zelltyp eine adulte Stammzelle, Progenitorzelle und/oder ein ausdifferenzierter Hautzelltyp immobilisiert.

"Stammzellen" sind Körperzellen, die noch nicht ausdifferenziert sind. Sie zeichnen sich durch ihre Fähigkeit zur Selbstreplikation und Differenzierung aus. Unterschieden werden zwei Formen: die embryonalen Stammzellen und die adulten Stammzellen. Zu den embryonalen Stammzellen zählen einerseits diejenigen, aus denen ein Embryo bis zu einem Stadium mit acht Zellen besteht. Aus diesen Zellen entwickeln sich später alle Zellformen des entstehenden Lebewesens. Sie werden totipotente Zellen genannt, zu denen ebenfalls die befruchtete Eizelle gehört. Andererseits sind bei den embryonalen Stammzellen jene aus den Blastozyten-Stadien einzuordnen, die als pluripotent bezeichnet werden. Aus ihnen können sich alle Arten von Körperzellen der Hauptgewebetypen differenzieren, jedoch keine Teile der Plazenta. Es wird hierbei weiterhin zwischen den eigentlichen embryonalen Stammzellen aus der inneren Zellmasse der Blastozyste, den EG-Zellen (Embryonal Gonad) und den EC-Zellen (Embryonal Carcinoma) unterschieden. Unter "adulten Stammzellen" sind dagegen jene nicht-ausdifferenzierten Körperzellen zu verstehen, die nach dem embryonalen Stadium entstehen und aus denen während der Lebensphase des Organismus spezialisierte Zellen gebildet werden. Aus adulten Stammzellen können durch mitotische Teilung weitere Stammzellen oder ausdifferenzierte Zellen hervorgehen. Obwohl embryonalen Stammzellen unterlegen, besitzen sie ein keimblattüberschreitendes Differenzierungspotential. Adulte Stammzellen sind in verschiedenen Organen, insbesondere in Knochenmark, Fettgewebe, Nabelschnur, Nabelschnurblut, Gehirn, Leber und Bauschspeicheldrüse, zu finden und in jedem Individuum verfügbar, so dass die Perspektive des Ersatzes durch körpereigene, d.h. autologe Zellen gegeben ist.

"Progenitorzelle" bezieht sich auf einen Abkömmling einer adulten Stammzelle. Sie weist hinsichtlich ihrer Regenerationsfähigkeit noch Stammzelleigenschaften auf, ist aber bereits auf einen künftigen Funktionsbereich festgelegt. Diese Festlegung ist jedoch umkehrbar, so dass Progenitorzellen pluripotent sind. Sie kommen vor allem im Knochenmark und in geringer Konzentration im Blutgefäßsystem vor. Auch die Dermatoblasten der Haut können als Progenitorzellen betrachtet werden.

Nach der Immobilisierung der Stammzellen und/oder Progenitorzellen können zügig zellspezifische Reaktionen eingeleitet werden, von denen insbesondere die Ausdifferenzierung erwähnt sei. Unter "Ausdifferenzierung" soll vorliegend die komplette phänotypische Ausprägung einer spezifischen Körperzelle aus einer Stammzelle und/oder Progenitorzelle verstanden werden. Die Ausdifferenzierung findet dabei nach der Immobilisierung in Schritt (a) und/oder Schritt (c) statt, das heißt, sie kann sich in einem eigenständigen Schritt unmittelbar an Schritt (a), (c) und/oder einen beliebigen nachfolgenden Schritt anschließen oder während eines oder mehrerer Schritte, die der Immobilisierung der Schritte (a) und/oder (c) folgen, durchgeführt werden. Bevorzugt ist die unmittelbare Ausdifferenzierung nach der Immobilisierung.

Die Verwendung von Stammzellen bietet die Vorteile, beliebige Phänotypen aus einem einzelnen ersten Zelltyp, einem einzelnen zweiten Zelltyp und/oder einem einzelnen weiteren Zelltyp zu erzeugen, so dass spezifische Zellschichten oder Organstrukturen aufgebaut werden. Darüber hinaus kann insbesondere durch Co-Immobilisierung und Co-Kultivierung von Stammzellen und/oder Progenitorzellen mit ausdifferenzierten Zellen eine Regenerationsfähigkeit der Hybridstruktur gewährleistet werden.

Vorzugsweise werden als Progenitorzellen basale Keratinozyten an der Hohlfasermembran und/oder an der bereits vorhandenen Hybridstruktur immobilisiert. Sie befinden sich in-vivo in der Regenerationsschicht (Stratum basale) der Epidermis und dienen der Hauterneuerung. Immobilisierte basale Keratinozyten können in-vitro zu Keratinozyten ausdifferenziert werden, deren Proliferation und Stratifizierung eine epidermale Struktur generiert.

In einer bevorzugten Ausführungsform der Erfindung werden Hautzelltypen immobilisiert, vorzugsweise Keratinozyten, Melanozyten, Merkelzellen, Basalzellen, Langerhanszellen und/oder Fibroblasten. Deren Rein- oder partielle Co-Kultivierung ist bisher hauptsächlich in Monolayer-Zellkulturen beschrieben, die für in-vitro Prüfungen, wie z. B. Phototoxizität oder Mutagenität, verwendet werden. Keratinozyten stellen hierbei den wesentlichen Baustein der Epidermis und Fibroblasten den wesentlichen Baustein der Dermis dar. Während Keratinozyten vorteilhaft zu einer mehrlagigen Struktur stratifiziert werden, die die organotypische in-vivo Situation widerspiegelt, wachsen Fibroblasten in dreidimensionalen Verbänden. Das erfindungsgemäße Verfahren bildet die Grundlage für die Erzeugung von mehrschichtigen, höheren Hautstrukturen, die als standardisierte Hauttransplantate oder als Hautsubstitute für dermatologische und pharmakologische Testreihen eingesetzt werden können. Als Hybridstruktur wird bevorzugt eine epidermale Struktur, eine dermale Struktur, oder eine epidermale und dermale Struktur erzeugt. Besonders bevorzugt wird eine Hybridstruktur aus Keratinozyten, eine Hybridstruktur aus Fibroblasten oder eine Hybridstruktur aus Keratinozyten und Fibroblasten. Sowohl das gleichzeitige Vorliegen einer epidermalen und einer dermalen Struktur als auch von Keratinozyten und Fibroblasten bedeutet deren Anordnung in getrennten Schichten, die nicht alternieren, vorzugsweise in zwei parallelen Schichten oder Polyschichten. Die Schichtabfolge in der erfindungsgemäßen Hybridstruktur orientiert sich am natürlichen Aufbau der Haut, indem sich die epidermale Struktur im distalen Bereich der Hybridstruktur und die dermale Struktur im proximalen Bereich der Hybridstruktur befindet. Dementsprechend werden Keratinozyten auf dem außen liegenden Bereich der Hohlfasermembran immobilisiert, kultiviert und gegebenenfalls stratifiziert. Proximal davon werden Fibroblasten entweder auf der innen liegenden Oberfläche oder auf der außen liegenden Oberfläche der Hohlfasermembran immobilisiert und kultiviert, wobei in letzterem Fall die Fibroblasten einen ersten Zelltyp und die Keratinozyten einen zweiten Zelltyp darstellen. In einer bevorzugten Ausgestaltung wird als Hybridstruktur eine epidermale und eine zwischen Hohlfasermembran und epidermaler Struktur angeordnete dermale Struktur erzeugt.

In einer weiteren Ausführungsform des vorliegenden Verfahrens werden die basalen Keratinozyten und/oder Keratinozyten in dynamischer Kultur über eine vierte Kultivierungsdauer stratifiziert. Die lineare Schichtbildung oder Stratifizierung dient der vorteilhaften Ausbildung einer dreidimensionalen epidermalen Struktur, die sich von innen nach außen aus Stratum basale, Stratum spinosum, Stratum granulosum, Stratum lucidum und Stratum corneum oder Teilen davon zusammensetzt. Im Stratum basale (Regenerationsschicht) erfolgt durch Zellteilung die Neubildung der Keratinozyten. Es dauert ca. 30 Tage bis ein neugebildeter Keratinozyt aus der Regenerationsschicht an die Hautoberfläche, also in die Hornschicht, gewandert ist. Im Stratum spinosum (Stachelzellschicht) sind die Keratinozyten durch bestimmte Strukturen, die Tonofibrillen, netzartig miteinander verbunden. In dieser Schicht kann es bei Hauterkrankungen zu Wasseransammlungen und damit zur Blasenbildung kommen. Die im Stratum granulosum (Körnerschicht) befindlichen Keratinozyten enthalten Keratohyalinkörner. Das Stratum lucidum (lichtbrechende Schicht) ist sehr schmal, so dass Zellgrenzen oder Zellkerne nicht mehr zu erkennen sind. Im Stratum corneum (Hornschicht) verbacken die aus den Keratinozyten hervorgegangenen Hornzellen mit Hornsubstanzen der Haut, z. B. Keratin, zu Hornschuppen, die dann abgestoßen werden.

Nach der Immobilisierung wird der zumindest eine erste Zelltyp, der vorliegend basale Keratinozyten und/oder Keratinozyten beinhaltet, zunächst bis zu einem konfluenten Zustand, das heißt einer geschlossenen Zellschicht, in einer ersten und optional dritten Kultivierungsdauer kultiviert. Während dieser Kultivierungsdauer(n) können die basalen Keratinozyten bereits ausdifferenziert werden. Anschließend wird das Medium abgelassen und die Stratifizierung über eine kontrollierte Begasung initiiert. Hierbei ist durch interzelluläre Adhäsionsfaktoren ein adhärentes Wachstum auf dem ursprünglichen Monolayer und den nachfolgenden Polyschichten gegeben. Die Geschwindigkeit und Dicke der Schichtenbildung in der vierten Kultivierungsdauer wird vorteilhaft über eine Sauerstoff/Kohlendioxid-Mischung kontrolliert. Nach Erreichen einer gewünschten Anzahl an Polyschichten kann die entstandene epidermale Struktur durch Zugabe von frischem Flüssigmedium in einer dritten Kultivierungsdauer gemäß Schritt (f) weiter kultiviert werden. Die Ausdifferenzierung basaler Keratinozyten ist auch erst unmittelbar vor, während oder nach dem Kultivierungsschritt (f) möglich.

Der gesamte Vorgang der Stratifizierung kann wiederholt werden, sofern der zumindest eine erste Zelltyp basale Keratinozyten und der zumindest eine zweite Zelltyp Keratinozyten beinhaltet oder umgekehrt. Diese Ausgestaltung kann auch entsprechend auf einen zweiten Zelltyp und einen weiteren Zelltyp angewendet werden.

Es ist nicht ausgeschlossen, dass die vierte Kultivierungsdauer mit einer oder allen anderen Kultivierungsdauern, vorliegend mit erster, zweiter und dritter Kultivierungsdauer bezeichnet, oder Teilen davon identisch ist. Bevorzugt ist eine eigenständige vierte Kultivierungsdauer für die Stratifizierung.

Des Weiteren ist eine Co-Stratifizierung realisierbar, indem neben den basalen Keratinozyten und/oder Keratinozyten mindestens ein weiterer erster Zelltyp und/oder mindestens ein weiterer zweiter Zelltyp immobilisiert, kultiviert und dann in die Polyschichten integriert wird. Vorzugsweise sind Endothelzellen für die Vaskularisation, Melanozyten für die Pigmentierung und/oder Langerhanszellen als Immunstimulans enthalten.

In einer weiteren bevorzugten Ausführungsform des vorliegenden Verfahrens werden in Schritt (a) zumindest zwei erste Zelltypen und/oder in Schritt (c) zumindest zwei zweite Zelltypen co-immobilisiert. Es ergeben sich vorliegend mehrere Varianten, zwei erste Zelltypen oder zwei zweite Zelltypen zu kombinieren: So können ein adulter Stammzelltyp und ein Progenitor-, Hautzell- oder Endothelzelltyp, ein Progenitorzelltyp und ein Hautzell- oder Endothelzelltyp, ein Hautzelltyp und ein Endothelzelltyp, zwei verschiedene Progenitorzelltypen oder zwei verschiedene Hautzelltypen immobilisiert werden. Vorzugsweise werden dabei als einer der zumindest zwei ersten Zelltypen und/oder einer der zumindest zwei zweiten Zelltypen Endothelzellen immobilisiert.

Das Endothel - auch einschichtiges Plattenepithel genannt - ist eine einschichtige Zellhaut, die Herzinnenräume, Blut- und Lymphgefäße von innen auskleidet. Dieses Deckgewebe besteht aus platten, flachkernigen und unregelmäßig begrenzten überlappenden Zellen, den "Endothelzellen". Das Endothel dient als Barriere zum Gewebe und produziert z. B. Stickstoffmonoxid, welches der Regulation des Tonus der Gefäßmuskulatur im Herz-KreislaufSystem dient. Daneben beeinflusst es die Fließfähigkeit des Blutes, u. a. durch Hemmung und Aktivierung von Gerinnungsprozessen. Ebenfalls spielen sich hier physiologische Entzündungs- und pathologische Allergiereaktionen ab. Die Inkorporation von Endothelzellen in die erfindungsgemäße Hybridstruktur ist folglich für die Analyse vorgenannter Prozesse ebenso vorteilhaft wie die Möglichkeit der inhärenten Nährstoffversorgung. Zur Stabilisierung der vaskulären Substruktur in der Hybridstruktur können die Endothelzellen zusätzlich mit Muskelzellen umgeben sein.

Wird die Hybridstruktur beispielsweise als Hauttransplantat verwendet, ist das Wachstum von Blutgefäßen des Wirts in das neu implantierte Biomaterial essentiell. Dieser Prozess kann vorteilhaft beschleunigt werden, wenn zuvor primäre Endothelzellen in die Hybridstruktur inkorporiert worden sind und durch stimulierende Substanzen die Ausbildung von Mikrokapillaren initiiert wird. Die Mikrokapillaren verbinden sich nach der Implantation mit der vorhanden Vaskulatur des Wirts. Es konnte gezeigt werden, dass Endothelzellen sowohl allein auch im co-kultivierten Zellverband auf Hohlfasermembranen erfolgreich wachsen und damit ein vaskuläres System aufbauen können.

Insbesondere das adhärente Wachstum auf der Hohlfasermembran ermöglicht auch eine Beschichtung auf ihrer außen liegenden Oberfläche mit einer endothelialen Struktur, auf der nachfolgend die mehrschichtige organotypische Hybridstruktur der Haut nach dem erfindungsgemäßen Verfahren erzeugt wird. Die Beschichtung der Hohlfasermembran mit Endothelzellen verläuft ähnlich den vorliegenden Verfahrensschritten (a) und (b), wobei in einem ersten Schritt Endothelzellen auf einer Hohlfasermembran immobilisiert und in einem zweiten Schritt die immobilisierten Endothelzellen in dynamischer Kultur über eine Kultivierungsdauer zu einer konfluenten Zellschicht kultiviert werden. Die vorherige Lehre der Erfindung und deren Ausführungsformen sind gültig und ohne Einschränkungen auf die Beschichtung der Hohlfasermembran mit Endothelzellen anwendbar, sofern es sinnvoll erscheint.

In einer weiteren bevorzugten Ausführungsform der Erfindung wird als Hybridstruktur eine epidermale und endotheliale Struktur, eine dermale und endotheliale Struktur, oder eine epidermale, dermale und endotheliale Struktur erzeugt, vorzugsweise eine epidermale und endotheliale Struktur, oder eine epidermale, dermale und endotheliale Struktur.

Der zumindest eine erste Zelltyp und optional zumindest eine zweite Zelltyp wird im erfindungsgemäßen Verfahren bevorzugt auf Hohlfasermembranen immobilisiert, die als Polymerhohlfasern ausgebildet sind. Die zugrunde liegenden Polymere können aus Polysulfon, Polyethersulfon, Polysulfonpolyvinylpyrollidinon, Polyamid, Acrylnitril, Polyacrylnitril, Polyetherimid, Polylactid, Polyglykolid, Polypeptid oder Derivaten davon bestehen, besonders bevorzugt aus Polyethersulfon, Polyetherimid, Polylactid oder Polyglycogen. Die synthetischen Polymere sind unkompliziert in Massenproduktion zu fertigen und können für spezifische Anwendungen modifiziert werden. Die Materialauswahl richtet sich nach der Verwendung der Hybridstruktur.

Bioabbaubare Materialien sind insbesondere für Implantate erwünscht. Nach Erfüllung ihrer primären Stütz- und Wachstumsfunktion soll die Matrix durch verschiedene Degradationsmechanismen, wie z. B. Hydrolyse, enzymatische Degradation und Dissoziation von Polymer-Polymer-Komplexen abgebaut werden. Solche biodegradierbaren Polymere sind beispielsweise Polylactide und Polyglykolide, die auf aliphatischen Polyestern basieren und sich bakteriell herstellen lassen. Die Degradation erfolgt hier hydrolytisch. Die physikalischen und chemischen Eigenschaften können vorteilhaft durch Variation von Lactid- und Glykolid-Anteilen verändert werden. Insbesondere die Abbauzeit kann auf diese Weise beeinflusst werden.

Dagegen sind nicht resorbierbare Materialien bevorzugt, wenn eine anhaltende Kultivierung der Hybridstruktur erwünscht ist, so dass eine stabile Gerüststruktur die organotypische Struktur realisiert. Insbesondere in-vitro Tests seien hier genannt. Aber auch ein problemloses und vollständiges Lösen und Entfernen der Hybridstruktur wird durch enzymatisch, chemisch oder physikalisch nicht degradierbare Polymermaterialien erleichtert. Bevorzugt wird der zumindest eine erste Zelltyp und/oder optional zweite Zelltyp auf Polymerhohlfasern aus Polyethersulfon immobilisiert. Das Material wird in einem Extrusionsverfahren unter Verwendung einer Polymerschmelze und superkritischem Kohlendioxid hergestellt. Hierbei kommt es zur Phasenumwandlung, ohne dass organische Lösungsmittel und eine thermische oder mechanische Behandlung notwendig sind. Die verwendete Polymerhohlfaser aus Polyethersulfon besitzt eine Porosität, die einen ausreichenden Gastransport und eine hinreichende Diffusion von Metaboliten gewährleistet und das Durchdringen der Poren mit ersten Zelltypen und/oder zweiten Zelltypen verhindert. In einer bevorzugten Ausgestaltung wird eine Hohlfasermembran mit einer Porengröße von höchstens 0,5 µm, vorzugsweise höchstens 0,1 µm, verwendet, so dass sowohl ein Zellwachstum in den Poren als auch eine Verbreitung durch die Poren in andere Kompartimente, das heißt vom Außenbereich ins Lumen oder umgekehrt, ausgeschlossen ist. Das dreidimensionale Gerüst aus Polyethersulfon bietet damit hervorragende Voraussetzungen für die Zellimmobilisierung und Kultivierung. Vor ihrem Einsatz werden die Polymerhohlfasern sterilisiert, wie z. B. in 70 % Ethanol und anschließend in Phosphat-gepuffertem Salin gewaschen.

In einer weiteren Ausführungsform des erfindungsgemäßen Verfahrens wird der zumindest eine erste Zelltyp ausschließlich auf der außen liegenden Oberfläche der Hohlfasermembran immobilisiert und kultiviert. Durch die Verwendung der porösen Hohlfasermembran wird eine Kompartimentierung des Bioreaktors in zwei Bereiche erreicht. Das erste Kompartiment wird durch den Außenbereich der Hohlfasermembran gebildet. In diesem Kompartiment erfolgt die Beimpfung des Bioreaktors mit mindestens einem ersten Zelltyp und die anschließende Kultivierung desselben auf der außen liegenden Oberfläche der Hohlfasermembran. Das zweite Kompartiment wird durch den inneren Bereich der Hohlfasermembran, das Lumen, dargestellt. Beide Kompartimente bilden zwei unabhängige Systeme und können separat mit verschiedenen Medien durchströmt werden. Insbesondere Epithelzellen, wie z. B. die Keratinozyten der Haut, können dadurch in einer Umgebung kultiviert werden, die natürlichen Bedingungen entspricht. Werden in folgenden Verfahrensschritten zumindest ein zweiter Zelltyp und gegebenenfalls zumindest ein weiterer Zelltyp in die Hybridstruktur integriert, sind diese vorzugsweise auf dem vorhandenen ersten Zelltyp adhärent anzusiedeln. Unter besonderen Gegebenheiten (z. B. spezifischen Wachstumsbedingungen), die vordergründig vom zweiten Zelltyp oder vom weiteren Zelltyp bestimmt werden, ist natürlich auch eine partielle oder vollständige Immobilisierung der Zelltypen auf der innen liegenden Oberfläche der Hohlfasermembran möglich.

Die dynamische Kultur im erfindungsgemäßen Verfahren wird also bevorzugt als Gradientenkultur durchgeführt. Flüssigmedium 1 wird kontinuierlich im Außenbereich zwischen der Hohlfasermembran und der Innenwand des Bioreaktors zugeführt, während das Flüssigmedium 2, dessen Zusammensetzung sich vorzugsweise vom Flüssigmedium 1 unterscheidet, das Lumen durchfließt. Außenbereich und Lumen stehen ausschließlich über die poröse Hohlfasermembran miteinander in Kontakt. Der Vorteil der Kompartimentierung liegt darin begründet, dass dem auf der außen liegenden Oberfläche befindlichen ersten Zelltyp zu definierten Zeitpunkten zusätzliche Medienkomponenten (Wachstumsfaktoren, Differenzierungsfaktoren, Sauerstoff-angereichertes Medium etc.) über das Lumen zugeführt werden kann. Toxische Metabolite, die sich negativ auf das Wachstum auswirken würden, werden gleichzeitig kontinuierlich ausgetragen. Im Lumen lassen sich außerdem hohe Volumenströme realisieren, um einen konvektiven Stofftransport zu ermöglichen, ohne einen negativen Einfluss auf die Zellen im Außenbereich zu nehmen. Das ist insbesondere im Fall scherkraftempfindlicher erster Zelltypen vorteilhaft.

Alternativ zur Gradientenkultur oder in Kombination mit selbiger kann die Perfusionskultur auch im Kreislauf erfolgen. Für die Kultivierung im Kreislauf ist die entsprechende Verschlauchung des Bioreaktors so zu wählen, dass Medienvorlage und Abfallvorlage einander entsprechen.

In einer weiteren Ausführungsform des erfindungsgemäßen Verfahrens wird der zumindest eine erste Zelltyp magnetisch immobilisiert, wobei gleichzeitig oder in beliebiger Reihenfolge an den ersten Zelltyp zumindest eine magnetisch-markierte Substanz gekoppelt wird, und die zumindest eine magnetisch-markierte Substanz an eine zumindest bereichsweise magnetische Hohlfasermembran gekoppelt wird.

Es wurde gefunden, dass Zellen, die mit einer magnetischen Markierung versehen sind, auf Zellträgern, vorzugsweise Hohlfasermembranen, aus einer Vielzahl an Materialien dauerhaft immobilisiert werden können, wenn diese Materialien in irgendeiner Form und zumindest bereichsweise mit magnetischen Eigenschaften verknüpft sind.

Im Sinne der Erfindung bedeutet "magnetisch", dass ein Magnetfeld vorhanden ist, das sowohl permanent durch einen Dauermagneten als auch zeitweilig, wie z. B. die Bewegung elektrischer Ladungen, erzeugt werden kann. Die magnetischen Eigenschaften von Festkörpern haben ihren Ursprung im Magnetismus der Atome/lonen und Elektronen, aus denen er aufgebaut ist. Es handelt sich dann um magnetisches Material, wenn die elementaren magnetischen Momente so ausgerichtet sind, dass sie sich nicht vollständig gegenseitig kompensieren, der Stoff also eine makroskopische Magnetisierung aufweist. Im Sinne der Erfindung soll "magnetisch" auch als Obergriff für die assozüerten Begriffe "magnetisierbar", "paramagnetisch" und "superparamagnetisch" verstanden werden, sofern sich aus dem konkreten Zusammenhang nicht ausdrücklich etwas anderes ergibt.

"Magnetisierbar" bezeichnet vorliegend die Eigenschaft eines Stoffes, insbesondere eines ferromagnetischen Stoffes, im Zuge der Einwirkung und des anschließenden Wegfalls eines externen Magnetfeldes eine verbleibende Magnetisierung oder Remanenz zu zeigen.

"Paramagnetisch" beinhaltet die Tendenz einer Substanz, in ein Magnetfeld hineinzuwandern. Paramagnetisches Verhalten findet man bei Substanzen mit ungepaarten Elektronen, da sich die Spinmomente der Elektronen parallel zum äußeren Magnetfeld ausrichten und es verstärken. Der Bahndrehimpuls der ungepaarten Elektronen steuert ebenfalls einen Anteil zum Paramagnetismus bei.

"Superparamagnetisch" weist auf eine besonders starke paramagnetische Tendenz hin.

Sowohl die magnetischen Eigenschaften der markierten Substanz als auch des Zellträgers, die im erfindungsgemäßen Verfahren zur Anwendung kommen, können durch magnetische Stoffe hervorgerufen werden, die vorzugsweise aus einer Gruppe ausgewählt sind, die keramische Oxidwerkstoffe, vorzugsweise Bariumoxid oder Eisenoxid, oder Metalle der VIII. Nebengruppe oder einer Legierung daraus, vorzugsweise Eisen, Kobalt, Nickel, deren Legierungen untereinander oder deren Legierungen mit Platin oder seltenen Erden, besonders bevorzugt eine Neodym-Eisen-Bor-Legierung oder eine Kobalt-Samarium-Legierung, enthält.

Bisher ist lediglich bekannt, dass mit der MACS-Technologie Zellen unter Verwendung magnetisch-markierter Antikörpern sortiert werden können. Die Technologie basiert auf so genannten MicroBeads, die an spezifische monoklonale oder polyklonale Antikörper gebunden sind. Die magnetische Markierung der Zellen in der MACS-Technologie kann entweder direkt mittels spezifischer MicroBead-gekoppelter Antikörper gegen eine spezifische Zelloberflächenstruktur erfolgen oder indirekt mittels primärer Antikörper gegen eine oder mehrere Zelloberflächenstrukturen, wobei die primären Antikörper von sekundären Antikörpern mit MicroBeads erkannt werden. Die markierten Zellen werden in einer Säulenmatrix, die sich in einem Magnetfeld befindet, zurückgehalten, während nicht-markierte Zellen aus der Säule eluiert werden. Nach dem Wegfall des Magnetfeldes werden die markierten Zellen aus der Säule gewonnen. Immobilisierung, Wachstum und/oder zellspezifische Prozesse auf magnetischen Oberflächen wurden bisher nicht beschrieben.

Das Immobilisierungsverfahren der vorliegenden Erfindung ist einfach und schnell durchzuführen sowie universell applikabel. Vorteilhaft ist die direkte Immobilisierung der Zellen auf dem Zellträger, die durch die Stärke des Magnetfeldes beeinflusst werden kann. Homogene Magnetfelder ermöglichen eine gleichmäßige Verteilung der Zellen auf der Oberfläche, wodurch die Zellen optimal mit Nährstoffen versorgt werden und zu einem geschlossenen Zellrasen ausbreiten. Die Analyse des Zellwachstums und zellspezifischer Reaktionen, wie z. B. Proliferation und Differenzierung, werden vereinfacht. Anstelle des Zusatzes unspezifischer Adhäsionsfaktoren wird die Anlagerung auf dem Zellträger durch mindestens eine magnetisch-markierte Substanz vermittelt. Dadurch wird nicht nur die gezielte Selektion und Immobilisierung einer Subpopulation von Zellen mit definierten Eigenschaften ermöglicht, sondern auch die Immobilisierung und nachfolgende Kultivierung der Zellen ohne Serum. Der Verzicht auf Serum und die darin enthaltenen extrazellulären Matrixglycoproteine als Adhäsionsfaktoren bedingt eine kostengünstige, risikofreie und reproduzierbare Fermentation mit höheren Ausbeuten, an die sich eine einfachere Aufarbeitung anschließt. Auch Optimierungsstrategien betreffend die Zellkultivierung sind leichter umzusetzen.

Sofern keine Einzelzellsuspension vorliegt, aber erwünscht ist, wird eine Probe, wie z. B. eine Gewebeprobe, zunächst durch mechanische und/oder proteolytische Prozesse in eine Einzelzellsuspension überführt. Die Einzelzellsuspension wird anschließend mit einer oder mehreren magnetisch-markierten Substanzen inkubiert. Darüber hinaus ist auch eine Inkubation von Zellverbänden, wie z. B. embryoiden Körpern oder Geweben, möglich.

Unter "magnetisch-markierten Substanzen" im Sinne der Erfindung versteht man Partikel oder Moleküle, die in der Lage sind, mit Zellen eine Assoziation einzugehen, wobei diese Assoziation nicht auf der magnetischen Markierung beruht.

Der Begriff der "Assoziation" bezieht sich hierbei auf jede Art der Wechselwirkung zwischen der magnetisch-markierten Substanz und der Zelle, insbesondere kovalente oder nicht-kovalente Bindungen, wie z. B. eine kovalente Bindung, hydrophobe/hydrophile Wechselwirkungen, van der Waals'sche Kräfte, Ionenbeziehung, Wasserstoffbrücken, Ligand-Rezeptor-Wechselwirkungen, Basenpaarungen von Nukleotiden oder Wechselwirkungen zwischen Epitop und Antikörper-Bindungsstelle sowie den Einschluss von Substanzen in die Zelle, wie z. B. durch Endocytose, Transformation oder Beschuss.

Magnetisch-markierte Substanzen umfassen vorzugsweise Biomoleküle, wie z. B. Proteine, Peptide und Nukleinsäuren, oder niedermolekulare organische Verbindungen. Die Markierung der Substanzen kann direkt, vorzugsweise über eine Chelat-Bindung oder eine kovalente Bindung an magnetische Stoffe, erfolgen. Die Markierung kann auch indirekt mittels einer chemischen Verbindung geschehen, die vorzugsweise kovalent an die Substanz gebunden ist. Die chemische Verbindung enthält dabei mindestens einen magnetischen Stoff, vorzugsweise über eine Chelat-Bindung, eine kovalente Bindung oder einen Einschluss desselben. Die Verwendung eines zusätzlichen Linkers ist ebenfalls möglich, wie z. B. in Form funktionalisierter Seitenketten von Magnetic Beads. Darüber hinaus sind auch magnetische Stoffe im isolierten Zustand oder im verkapselten Zustand, wie z. B. Magnetic Beads, als magnetisch-markierte Substanzen im Sinne der Erfindung aufzufassen.

Die Bezeichnung "Magnetic Bead" charakterisiert Partikel, wie z. B. Polystyrol-Kügelchen, die mindestens einen magnetischen Stoff einschließen, vorzugsweise einen paramagnetischen oder superparamagnetischen Stoff, wie z. B. Magnetit oder Ferrit. Die Partikel besitzen funktionalisierte Oberflächen mit Seitenketten, die beispielsweise Amin-, Carboxy-, Aldehyd-, Epoxy-, Iminodiacetat-, Hydrazid-, NADPA- oder andere Gruppen tragen, und darüber eine Kopplung an andere Moleküle erlauben.

Unter einer "Inkubation" im Sinne der Erfindung versteht man eine Kontaktierung von mindestens zwei Substanzen, die mit oder ohne chemische Konvertierung erfolgen kann. Eine der Substanzen ist hierbei die magnetisch-markierte Substanz.

Magnetisch-markierte Substanzen können sich zum einen in Lösung befinden, so dass sie insbesondere durch Diffusion an die Zelloberfläche gelangen und an die Zellen gekoppelt werden. Bevorzugt werden hierbei exponierte Oberflächenproteine kontaktiert. Die Lösung umfasst geeignete Komponenten, die die Überlebensfähigkeit der Zellen sichern, wie z. B. C-Quelle, N-Quelle, Mineralien, Puffer etc. Vorzugsweise handelt es sich um eine Nährlösung oder eine physiologische Kochsalzlösung. Entsprechende Lösungen sind dem Fachmann bekannt. Nach einer definierten Inkubationszeit von magnetisch-markierten Substanzen und Zellsuspension, die hauptsächlich von der Art der markierten-spezifischen Substanzen abhängt, werden die Substanz-markierten Zellen mit dem zumindest partiell magnetischen Zellträger in Kontakt gebracht und die Zellen über die magnetisch-markierten Substanzen an den Zellträger immobilisiert. Wiederum sind Diffusionsprozesse für den Stofftransport, vorliegend den Transport der Substanz-markierten Zellen in Lösung an die feste Oberfläche des Zellträgers, verantwortlich. Nicht-markierte Zellen der Zellsuspension verbleiben ungebunden in Lösung und können in Waschschritten entfernt werden.

Zum anderen kann zumindest eine magnetisch-markierte Substanz in einem ersten Schritt über magnetische Wechselwirkungen auf einem Zellträger mit magnetischen Eigenschaften fixiert werden, indem der Zellträger mit der magnetisch-markierten Substanz kontaktiert wird und ungebundene Moleküle der Substanz in Waschschritten entfernt werden. Hierfür werden insbesondere Pufferlösungen verwendet, die eine die magnetisch-markierte Substanz stabilisierende Umgebung gewährleisten. Der Fachmann ist mit der Auswahl passender Lösungen in Abhängigkeit von der verwendeten Substanz und ihrer Herstellung vertraut. Die Inkubationszeit wird wesentlich vom vorhandenen Magnetfeld bestimmt. Anschließend wird der mit der magnetisch-markierten Substanz beschichtete Zellträger mit der Zellsuspension inkubiert. Die diffusionskontrollierte Markierung mit der magnetischen Substanz geht mit der Immobilisierung der Zellen einher.

In einer dritten Variante der Verfahrensabfolge können die Schritte auch gleichzeitig durchgeführt werden. Die verwendete Lösung muss zugleich Zellvitalität und Substanzstabilität gewährleisten. Die Inkubationszeit ist verlängert, vorzugsweise verdoppelt. Diffusionsfördernde Maßnahmen, wie z. B. Schütteln, Rühren oder Durchströmen, können sich jedoch auf die Kopplungen der Schritte beschleunigend auswirken. Im Anschluss an die gemeinsame Inkubation von Zellen, magnetisch-markierter Substanz und Zellträger werden ungebundene Bestandteile in Waschschritten entfernt.

Bevorzugt ist die Inkubation der Zellen mit mindestens einer magnetisch-markierten Substanz in Lösung, wodurch die entscheidende Selektion von Zellen, insbesondere einer Subpopulation innerhalb einer Einzelzellsuspension, effizient im Volumen der Lösung durchgeführt werden kann und die magnetische Anziehung zwischen Substanz-markierten Zellen und Zellträger eine rasche Immobilisierung auf der Oberfläche gewährleistet.

Für eine gleichmäßige Verteilung der Zellen auf dem Zellträger ist es vorteilhaft, den Prozess der Immobilisierung so durchzuführen, dass sich Zellen und Zellträger in einer Lösung befinden, indem beispielsweise der Zellträger in die Zellsuspension eingebracht wird, wie z. B. durch Eintauchen, Umspülen etc.

Die immobilisierten Zellen werden anschließend in einer geeigneten Nährlösung kultiviert, bis eine gewünschte Zelldichte erreicht ist, oder zellspezifische Reaktionen eingeleitet werden.

Voraussetzung für die Immobilisierung der Zellen im erfindungsgemäßen Verfahren ist, dass der Zellträger zumindest bereichsweise mit magnetischen Eigenschaften versehen wird. Die magnetischen Eigenschaften können inhärenter Bestandteil des Materials des Zellträgers, was auch eine nachträgliche Modifikation des zunächst nicht-magnetischen Materials einschließen soll, oder durch mindestens einen externen magnetischen Stoff hervorgerufen sein. Im Fall der letztgenannten räumlichen Trennung wird der magnetische Stoff im Sinne der Erfindung so platziert, dass der Zellträger zwischen der Zellsuspension und dem magnetischen Stoff lokalisiert ist. Für die vorteilhafte Ausgestaltung des Zellträgers als Flachmembran bedeutet das insbesondere die Integration des magnetischen Stoffes in das Kulturgefäß und eine zweidimensionale Immobilisierung. In der vorteilhaften Ausgestaltung des Zellträgers als Hohlfaser befindet sich der magnetische Stoff, wie z. B. in Form eines Ringmagneten, im Inneren derselben. Das vom magnetischen Stoff hervorgerufene Magnetfeld bedingt die Wanderung magnetisch-markierter Substanzen respektive Substanz-markierter Zellen an die Oberfläche des Zellträgers und deren Immobilisierung.

Der Zellträger kann auch zumindest bereichsweise elektromagnetisch magnetisiert sein. Dabei wird der Zellträger durch einen externen stromdurchflossenen Leiter entweder selbst magnetisiert oder das Magnetfeld des Leiters, das sich über den Zellträger erstreckt, wird als magnetische Eigenschaft des Zellträgers interpretiert. Des Weiteren kann der Zellträger aus einem elektrisch-leitenden Material bestehen und im stromdurchflossenen Zustand ein eigenes Magnetfeld aufbauen. Der Vorteil des Elektromagnetismus im vorliegenden Verfahren besteht in der genauen Steuerung des Magnetfeldes und der Möglichkeit des Abschaltens des Magnetfeldes. Die erfindungsgemäße magnetische Kopplung kann insbesondere zur Initiation der Immobilisierung, gefolgt von einem adhärenten Zellwachstum, genutzt werden, oder erlaubt das Ablösen der immobilisierten und ggf. kultivierten Zellen oder höherer Zellschichten, was beispielsweise durch eine Umkehr der Stromrichtung und damit eine Umpolung des Magnetfeldes unterstützt wird.

Bevorzugt wird im erfindungsgemäßen Verfahren mindestens ein magnetischer Stoff in den Zellträger eingebracht. Die Modifikation des Zellträgers mit dem magnetischen Stoff kann dabei aus einer Substanz- oder einer Oberflächenmodifizierung resultieren. Im Fall der bevorzugten Substanzmodifikation wird beispielsweise ein Material für die Herstellung des Zellträgers verwendet, welches einen entsprechenden magnetischen Stoff aufweist oder welchem ein magnetischer Stoff zugemischt ist (Volumenmodifikation). Die Generierung magnetischer Eigenschaften durch Volumen modifikation kann vorzugsweise während des Herstellungsverfahrens, aber auch im Zuge einer nachträglichen Diffusion erfolgen. Der magnetische Stoff liegt vorzugsweise als Partikel vor, die durch eine geometrische Form und Größe charakterisiert sind, wobei es sich besonders bevorzugt um kugelförmige Partikel handelt, ganz besonders bevorzugt um kugelförmige Partikel mit einem Durchmesser von 0,01 µm bis 1 µm. Die Partikel werden beispielsweise im Extrusionsverfahren einer Polymerschmelze beigemengt und beim Aushärten in einer statistischen Verteilung in das polymere Material inkludiert. Sowohl die Verwendung magnetischer oder magnetisierter, vorzugsweise ferromagnetischer Stoffe, als auch das Einbringen magnetisierbarer Stoffe und die anschlie-βende Magnetisierung im Zellträger, entweder durch ein externes Magnetfeld oder das Magnetfeld der magnetisch-markierten Substanzen, sind denkbar. Es ist ebenfalls nicht ausgeschlossen, dass das Material des gesamten Zellträgers magnetisch ist.

Inhärente magnetische Eigenschaften des Zellträgers können auch durch eine nachträgliche Oberflächenmodifizierung erzeugt werden. Hierbei wird die Oberfläche des Zellträgers mit einer magnetischen Funktion ausgestattet. Zum einen kann eine kovalente Oberflächenfunktionalisierung mit dem magnetischen Stoff selbst oder den magnetischen Stoff enthaltenen Verbindungen, ggf. über einen Linker, durchgeführt werden, wie z. B. durch nasschemische Modifizierung oder Pfropfung. Vorzugsweise werden Magnetic Beads über funktionalisierte Seitenketten, deren Auswahl in Abhängigkeit vom Zellträger erfolgt, gebunden. Zum anderen ist eine nicht-kovalente Oberflächenfunktionalisierung machbar, wie z. B. durch Chelat-Bindung von magnetischen Stoffen, vorzugsweise ferromagnetischen Kationen, an entsprechende Gruppen, wie z. B. Iminodiacetat, oder durch die Beschichtung des Zellträgers mit einem magnetischen Stoff, vorzugsweise einer Legierung.

Denkbar ist aber auch die Verwendung eines Zellträgers, der sowohl substanz- als auch oberflächenmagnetisiert ist.

Es ergeben sich vorliegend drei Varianten, die magnetischen Eigenschaften von Zellträger und magnetisch-markierter Substanz zu kombinieren, wobei nachfolgend "magnetisch" nur in seiner klassischen Definition im Sinne der Erfindung und nicht als Oberbegriff zu betrachten ist: 1. Sowohl Zellträger als auch Substanz sind magnetisch markiert. 2. Der Zellträger ist magnetisch, während die Substanz magnetisierbar, paramagnetisch oder superparamagnetisch markiert sind. 3. Der Zellträger ist magnetisierbar, paramagnetisch oder superparamagnetisch, während die Substanz magnetisch markiert sind. Bevorzugt ist die zweite Variante, um die gegenseitige Anziehung und Verklumpung von magnetisch-markierten Substanzen zu unterbinden.

In einer Ausführungsform der vorliegenden Erfindung werden als magnetische Stoffe keramische Oxidwerkstoffe in den Zellträger eingebracht, vorzugsweise Bariumoxid oder Eisenoxid. In einer weiteren Ausführungsform der Erfindung werden ein Metall der VIII. Nebengruppe oder eine Legierung daraus in den Zellträger eingebracht, vorzugsweise Eisen, Kobalt, Nickel, deren Legierungen untereinander oder deren Legierungen mit Platin oder seltenen Erden, besonders bevorzugt Neodym-Eisen-Bor-Legierungen oder Kobalt-Samarium-Legierungen.

In einer bevorzugten Ausführungsform der Erfindung wird der zumindest eine magnetische Stoff in einem Muster in den Zellträger eingebracht. Diese magnetische Funktionalisierung der Zellträgerstruktur ermöglicht, die Zellen in einem adäquaten Muster auf der Oberfläche zu lokalisieren. Die gewünschten Zellen können damit in einem genau definiertem Abstand auf der Oberfläche des Zellträgers immobilisiert werden. Insbesondere der Abstand der Zellen ist entscheidend für das Wachstumsverhalten. Magnetische Muster im Zellträger bedingen vorteilhaft, dass die Zellen nicht als Zellaggregate, sondern vorzugsweise als Einzelzellen immobilisiert werden. Dadurch wird eine sehr gleichmäßige Immobilisierung in einer zunächst lückenbehafteten Monoschicht erreicht. Die Monoschicht kann durch nachfolgendes adhärentes Zellwachstum mittels einer Expression von Zelladhäsionsmolekülen, wie z. B. E-Selectin und/oder ICAM-1, konfluent werden und in Polyschichten übergehen. Die erzielte Zelldichte ist dabei vorzugsweise über den gesamten Zellträger konstant.

In einer besonders bevorzugten Ausführungsform der Erfindung wird das Muster als Raster, Linien oder Spots ausgebildet. In einer ganz besonders bevorzugten Ausführungsform der Erfindung wird das Muster in einer regelmäßigen Anordnung in den Zellträger eingebracht, das heißt, das Muster wiederholt sich in räumlich konstanten Intervallen. Zur Erzeugung der Muster sind beispielsweise magnetische Maschennetze denkbar. Der bevorzugte definierte Abstand der immobilisierten Einzelzellen beträgt zwischen 10 µm und 200 µm, vorzugsweise zwischen 50 µm und 100 µm.

Für die Ansiedlung von Zellen hat es sich zudem als vorteilhaft erwiesen, wenn der Zellträger eine plastische Oberflächenstruktur aufweist, die beispielsweise durch nachträgliche Prägung des Zellträgers oder bereits während des Herstellungsprozesses des Zellträgers erzeugt werden kann.

In einer Ausführungsform der vorliegenden Erfindung wird als magnetisch-markierte Substanz mindestens ein zell-spezifisches Molekül eingesetzt. Zell-spezifische Moleküle ermöglichen die Selektion und Markierung einer bestimmten Zellpopulation mit definierten Eigenschaften und damit die vorteilhafte Immobilisierung einer reinen Zellkultur. Darüber hinaus können aber auch Co-Kulturen durch die Verwendung mehrerer zell-spezifischer Moleküle oder eines zell-unspezifischen Moleküls immobilisiert werden, wobei die Verteilung auf dem Zellträger statistisch erfolgt. Bevorzugt ist die Immobilisierung von reinen Zellkulturen.

Unter "zell-spezifischen Molekülen" im Sinne der Erfindung versteht man hochaffine Moleküle, vorzugsweise Proteine, Peptide, Nukleinsäuren, niedermolekulare Liganden und andere Moleküle mit einer Affinität zu molekularen Zellbestandteilen.

Die Proteine und Peptide sind vorzugsweise ausgewählt aus der Gruppe der Antikörper, Rezeptoren, Lektine, Avidine, Lipoproteine, Glycoproteine, Oligopeptide, Peptid-Liganden und Peptid-Hormone. Besonders bevorzugt sind Antikörper, die beispielsweise an freien NH₂-Gruppen mit Carboxy-terminierten, Epoxy-aktivierten oder Aldehyd-modifizierten Magnetic Beads markiert werden können. Des Weiteren ist die Kopplung von Antikörpern an Amin-terminierte, wie z. B. mit DADPA, oder Hydrazid-modifizierte Magnetic Beads realisierbar.

Als "Antikörper" wird ein Polypeptid bezeichnet, das durch ein Immunoglobulin-Gen oder ein Fragment davon kodiert wird. Entsprechend der Erfindung können Antikörper als intakte Immunoglobuline oder eine Anzahl gut charakterisierter Fragmente vorhanden sein. Die Fragmente sind vorzugsweise ausgewählt aus der Gruppe der Fab-Fragmente, Fc-Fragmente, Einzelstrang-Antikörper (scFv), variablen Regionen, konstanten Regionen, H-Ketten (V_{H}) und L-Ketten (V_{L}), besonders bevorzugt aus den Fab-Fragmenten und scFv. Fragmente, wie z. B. Fab-Fragmente und Fc-Fragmente, können durch Spaltung mit verschiedenen Peptidasen erhalten oder rekombinant exprimiert werden, vorzugsweise scFv. Intakte polyklonale Antikörper können in einem Säugetierorganismus gebildet werden, wenn das Antigen eine Immunreaktion verursacht. Hierzu muss das Antigen dem Organismus fremd sein und ein Molekulargewicht größer 3000 g/mol haben. Darüber hinaus sind gängige Techniken zur Erzeugung monoklonaler Antikörper, wie die Hybridoma-Technologie, dem Fachmann bekannt.

Bevorzugte Nukleinsäuren im erfindungsgemäßen Verfahren sind einzelsträngige oder doppelsträngige DNA oder RNA, Oligonukleotide, Aptamere oder Spiegelmere, besonders bevorzugt sind Aptamere und Spiegelmere. Aptamere zeigen eine hohe Affinität für eine große Vielfalt an so genannten Target-Molekülen, die Proteine, Peptide und kleine Moleküle umfassen können, wie z. B. organische Farbstoffe, Nukleotide, Aminosäuren, Vitamine, Alkaloide etc. Damit ist ein den Antikörpern vergleichbares Einsatzspektrum gegeben. Insbesondere die 2'-Hydroxyl-Gruppe in RNA fördert eine Vielzahl von intra- und intermolekularen Kontakten, wobei die letztgenannten Kontakte zwischen Molekülen der gleichen Sequenz oder mit verschiedenen Sequenzen oder zwischen RNA und beliebigen anderen Molekülen, die nicht aus RNA bestehen, auftreten können. Diese Nukleinsäure-Liganden werden durch einen effizienten in-vitro Selektionsprozess identifiziert, der als SELEX (Systematic Evolution of Ligands by Exponential Enrichment) bezeichnet wird. Aufgrund der Sensibilität von RNA gegenüber einem nukleolytischen Abbau in biologischen Lösungen werden RNA-Aptamere insbesondere modifiziert, wie z. B. mit Phosphorothioaten oder als Spiegelmere, synthetisiert und eingesetzt. Spiegelmere, das heißt die L-RNA-Konformation der Aptamere, widerstehen einer natürlichen Degradation über lange Zeiträume.

Aptamere können durch Phosphoramidit-Chemie synthetisiert werden. Außerdem ist es möglich, längere Aptamere mit beispielsweise mehr als 30 Nukleotiden durch in-vitro Transkription kostengünstig und in großen Ausbeuten herzustellen. Selektion, Synthese und Aufreinigung von Aptameren sind dem Fachmann bekannt.

Aptamere können an Magnetic Beads gebunden werden, die z. B. Carboxy-terminierte oder Epoxy-aktivierte Seitenketten tragen. Des Weiteren ist eine Kopplung von Magnetic Beads an die Ribose bzw. Desoxyribose der Aptamere, wie z. B. über eine Hydrazon-Bindung, möglich.

Biotin, Biotin-Derivate und Steroide stellen bevorzugte Beispiele für niedermolekulare Liganden im erfindungsgemäßen Verfahren dar.

Resultierende Ligand-Akzeptor-Komplexe aus spezifischer Substanz und molekularem Zellbestandteil sind vorzugsweise Antikörper und Antigen, Aptamer und Target, sowie Oligonukleotid und Plasmid-DNA.

Erfindungsgemäß kann die Markierung der Zellen auch unspezifisch erfolgen, wobei entweder von einer Reinkultur der Einzelzellsuspension ausgegangen wird oder die Immobilisierung einer Mischkultur gewollt ist. Beispielsweise können Magnetic Beads über vorhandene funktionalisierte Seitenketten kovalent an Oberflächenstrukturen von Zellen gekoppelt werden.

Als weitere unspezifische Methode der Zellmarkierung kann eine magnetisch-markierte Substanz in Zellen eingeschlossen werden. Die Aufnahme kann beispielsweise durch Beschuss der Zellen mit Magnetpartikeln geschehen, vorzugsweise mit paramagnetischen oder superparamagnetischen Stoffen, die auch umhüllt sein können (wie z. B. Magnetic Beads). Als Nanopartikel sind so genannte Quantenpunkte (Quantum Dots) für die Inkorporation in Zellen durch Beschuss prädestiniert, deren sphärische Kristalle aus Halbleitermaterialien im Größenbereich von 5 nm bis 100 nm liegen. Die geringe Größe erfordert ein starkes Magnetfeld. Weiterhin kann die Inkorporation von magnetischen Stoffen im Sinne der Erfindung durch Endocytose initiiert werden, wobei die Umhüllung fester Magnetpartikel mit einer Erkennungsstruktur essentiell ist, welche die partikuläre Aufnahme in die Zelle (Phagocytose) fördert. Ähnliches gilt für die Pinocytose. Darüber hinaus ist die Transformation von magnetisch-markierten Plasmiden oder Oligonukleotiden, die mit Magnetic Beads gekoppelt sind, denkbar. Die skizzierten Methoden der unspezifischen Aufnahme von Partikeln in Zellen sind dem Fachmann bekannt.

Die Substanz-markierten Zellen werden im erfindungsgemäßen Verfahren vorzugsweise auf Zellträgern immobilisiert, die als Polymerflachmembran, Polymerhohlfaser oder Polymerpartikeln ausgebildet sind. Die zugrunde liegenden Polymere können aus Polysulfon, Polyethersulfon (PES), Polysulfonpolyvinylpyrollidinon, Polyamid, Acrylnitril, Polyacrylnitril, Polyetherimid, Polylactid, Polyglycogen, Polypeptid oder Derivaten davon bestehen, vorzugsweise aus Polyethersulfon. Die synthetischen Polymere sind unkompliziert in Massenproduktion zu fertigen und können für spezifische Anwendungen modifiziert werden. Die Mehrzahl dieser Polymere zeichnet sich ebenfalls dadurch aus, dass sie biologisch, insbesondere enzymatisch, nicht resorbierbar sind.

In einer bevorzugten Ausführungsform der vorliegenden Erfindung werden die Zellen auf Polymerhohlfasern aus Polyethersulfon immobilisiert. Polyethersulfonfasern können beispielsweise als kontinuierliche Hohlfäden um einen Zylinder gewickelt werden, mit einem inneren Durchmesser (Hohlkern) von ca. 1 mm. Die Porendurchmesser liegen bei etwa 100 nm oder weniger. Das dreidimensionale Gerüst aus Polyethersulfon bietet hervorragende Voraussetzungen, um Zellen zu immobilisieren und zu kultivieren.

Das erfindungsgemäße Verfahren eignet sich zur Immobilisierung einer Vielzahl von Zellen und Zelllinien verschiedener Gewebe und Zelltypen, wie z. B. Endothelzellen, Epithelzellen, Glialzellen, Osteoblasten, Hautzellen, Leberzellen oder Nierenzellen. Die Zellen können anhand spezifischer Markermoleküle gezielt mit magnetisch-markierten Substanzen aus Zellsuspensionen gefischt und fixiert werden. Deren anschließendes Wachstum ist insbesondere in Perfusionskulturen erwünscht, die sich durch eine kontrollierte Versorgung der Zellen mit Nährstoffen und Sauerstoff auszeichnen. Hierfür ist sowohl eine feste Haftung der Zellen innerhalb des durchströmten Bioreaktors, die ein Auswaschen verhindert, als auch eine minimale Zellkontaktfläche, die Stoffwechselvorgänge und Stofftransport wenig beeinflusst, nötig. Diesen Anforderungen wird die magnetische Kopplung der vorliegenden Erfindung gerecht, wobei die Zellen vorzugsweise auf Hohlfasern im Perfusionsreaktor fixiert werden. Darüber hinaus können mit dem bereitgestellten Verfahren auch schwierig an Träger zu koppelnde Zellen immobilisiert werden. Insbesondere Endothelzellen zeigen eine schlechte Adhäsion und Verbreitung auf Hohlfasern, die aus den gleichen Polymersystemen, die zur Formierung von Polymerflachmembranen eingesetzt werden, bestehen.

In einer bevorzugten Ausführungsform der Erfindung werden Hautzellen immobilisiert, vorzugsweise Keratinozyten, Melanozyten, Merkelzellen, Endothelzellen und/oder Fibroblasten, besonders bevorzugt Keratinozyten, Fibroblasten und/oder mikrovaskuläre Endothelzellen. Das erfindungsgemäße Verfahren bildet nunmehr die Grundlage für die Erzeugung von mehrschichtigen, höheren Hautstrukturen, die als Hauttransplantate oder als Hautsubstitute für dermatologische und pharmakologische Testreihen eingesetzt werden können. Hierfür wird beispielsweise eine Subpopulation Keratinozyten durch magnetische Kopplung selektiv und fest an den Zellträger immobilisiert und bis zu einem konfluenten Zustand der Zellen, d. h. einer geschlossene Zellschicht, kultiviert. Dem schließt sich die Stratifizierung der Keratinozyten zu einer neoepidermalen Struktur an. Bevorzugt ist die Kultivierung der Hautzellen in dreidimensionalen Gerüststrukturen, besonders bevorzugt Hohlfasern, in einer so genannten organotypischen Zellkultur, die die in-vivo Situation widerspiegelt. Innerhalb der Hohlfasern können insbesondere Fibroblasten, vorzugsweise humanen Ursprungs, und/oder mikrovaskuläre Endothelzellen immobilisiert werden, wobei eine Co-Immobilisierung für die Vaskularisation der dermalen Struktur bevorzugt ist.

In einer anderen bevorzugten Ausführungsform der vorliegenden Erfindung werden Stammzellen oder Progenitorzellen immobilisiert. Die Subpopulation an Stammzellen und/oder Progenitorzellen in einer Gewebeprobe ist sehr gering. Während in herkömmlichen Immobilisierungsverfahren die Begegnung der Zelle mit dem Zellträger dem Zufall überlassen und damit bei geringer Dichte der Zielzellen die Kontaktierung mit dem Zellträger zeitaufwendig ist, übt das Magnetfeld im vorliegenden Verfahren eine gerichtete Kraft auf Substanz-markierte Zellen aus. Spezifische Markermoleküle, wie z. B. Oberflächenproteine, machen Stammzellen oder Progenitorzellen für die magnetisch-markierten Substanzen erkenntlich. Daher ist es möglich, viele Zellen innerhalb kurzer Zeit auf dem Zellträger zu immobilisieren. Zellstress und suboptimale Kultivierungsbedingungen während der Immobilisierung werden auf ein Minimum reduziert. Nachfolgend können zügig zellspezifische Reaktionen eingeleitet werden, von denen insbesondere die Differenzierung erwähnt sei. Sie ermöglicht die Erzeugung spezifischer Zellschichten oder Organstrukturen, vorzugsweise oben genannter Hautstrukturen. In einer besonders bevorzugten Ausführungsform der Erfindung werden deshalb als adulte Stammzellen in der Basalschicht befindliche Keratinozyten an dem Zellträger immobilisiert.

In einer weiteren Ausgestaltung der Erfindung werden Zellen einer Einzelzellsuspension, einer Subpopulation der Einzelzellsuspension oder eines Zellverbandes gekoppelt, vorzugsweise embryoide Körper.

Embryonale Stammzellen (ES-Zellen) können in Anwesenheit des "cytokine leukemia inhibiting factor" (LIF) in einem undifferenzierten Stadium gehalten werden. Unter "embryoiden Körpern" versteht man Strukturen, die in einem ersten Aggregationsschritt aus ES-Zellen, die das undifferenzierte Stadium verlassen, geformt werden und sich unter geeigneten Bedingungen zum reifen embryonalen Gewebe ausdifferenzieren. Hierzu werden die Zellen in einer Suspensionskultur in Spinnerkulturgefäßen kultiviert.

Gegenstand der Erfindung ist auch eine Hybridstruktur, die
(a) einen zumindest bereichsweise magnetischen Zellträger,
(b) zumindest eine an einer Oberfläche des Zellträgers gekoppelte magnetisch-markierte Substanz, und
(c) an die zumindest eine magnetisch-markierte Substanz gekoppelte Zellen
umfasst.

In noch einer weiteren Ausgestaltung des erfindungsgemäßen Verfahrens wird die Dichte der Zellstruktur durch die Messung des transzellulären Widerstands über die Hohlfasermembran in einer Messzelle kontrolliert, wobei die Messzelle aus einer Gegenelektrode im Lumen der Hohlfasermembran und einer Ringblechelektrode im Außenbereich der Hohlfasermembran, die beide mit einem Widerstandsmessgerät verbunden sind, und Elektrolytlösung im Lumen und Außenbereich besteht. Die Messung des transzellulären Widerstands basiert auf dem Vergleich der Widerstandsmesswerte der juvenilen Hohlfasermembran und der Widerstandswerte der mit Zellen besiedelten Hohlfasermembran. Dank der Elektrodenanordnung erfolgt die Widerstandsmessung in einem wohldefinierten zentralsymmetrischen Feld. Die Messung zeichnet sich durch eine hohe Genauigkeit aus, so dass die Reifung des Hautgewebes optimal kontrolliert und standardisiert werden kann.

Gegenstand der Erfindung ist auch eine organotypische Hybridstruktur, die nach einem Verfahren erzeugt ist, das nachfolgende Schritten umfasst:
(a) Immobilisierung von zumindest einem ersten Zelltyp auf einer Hohlfasermembran, und
(b) Kultivierung des zumindest einen ersten Zelltyps in dynamischer Kultur über eine erste Kultivierungsdauer zu einer mehrschichtigen organotypischen Hybridstruktur der Haut.

Die vorherige Lehre des erfindungsgemäßen Verfahrens und deren Ausführungsformen sind gültig und ohne Einschränkungen auf die organotypische Hybridstruktur der Haut anwendbar, sofern es sinnvoll erscheint.

Ein weiterer Gegenstand der Erfindung ist eine organotypische Hybridstruktur, umfassend
(a) eine Hohlfasermembran mit einer innen liegenden Oberfläche und einer außen liegenden Oberfläche, und
(b) eine zumindest auf der außen liegenden Oberfläche angeordnete mehrschichtige Gewebestruktur aus zumindest einem ersten Zelltyp.

In einer Ausgestaltung der organotypischen Hybridstruktur weist die Gewebestruktur eine epidermale Struktur aus Keratinozyten als ersten Zelltyp auf. In einer weiteren Ausgestaltung der organotypischen Hybridstruktur enthält die Gewebestruktur zumindest einen zweiten Zelltyp und optional zumindest einen weiteren Zelltyp. In einer bevorzugten Ausgestaltung der organotypischen Hybridstruktur weist die Gewebestruktur eine dermale Struktur aus Fibroblasten als zweiten Zelltyp auf, wobei die dermale Struktur zwischen der außen liegenden Oberfläche und der epidermalen Struktur angeordnet ist. In einer anderen bevorzugten Ausgestaltung der organotypischen Hybridstruktur weist die Gewebestruktur eine endotheliale Struktur aus Endothelzellen als weiteren Zelltyp auf, wobei die endotheliale Struktur auf der innen liegenden Oberfläche angeordnet ist.

Noch ein weiterer Gegenstand der Erfindung ist die Verwendung der organotypischen Hybridstruktur für in-vitro Toxizitätstests. Das vorliegende Hautäquivalent kann international standardisierte Tierversuche, die nach den Vorschriften der EU und den Prüfmethoden der OECD durchgeführt werden, vollständig ersetzen. Insbesondere kann die erfindungsgemäße Hybridstruktur der Haut der im Februar 2003 in Kraft getretenen 7. Änderungsrichtlinie zur Kosmetikrichtlinie von 1976 genügen, die in einzelnen Schritten zu einem Verbot der Vermarktung von Kosmetika führen soll, deren Inhaltsstoffe in Tierversuchen geprüft wurden. Darüber hinaus ist selbstverständlich der Austausch schlechterer in-vitro Hautmodelle, wie sie bereits in einfachen und schnellen in-vitro Prüfungen Anwendung finden, gegeben, da sich mit dem vorliegenden Hautäquivalent die Möglichkeit eröffnet, komplexe physiologische Prozesse zu analysieren. Die Hybridstruktur kann auch bei Substanztests in der Pharma-und Biotechnologieindustrie eingesetzt werden.

Der Test wird im Bioreaktor durchgeführt, in dem das Hautäquivalent der zu untersuchenden Substanz ausgesetzt ist. Die Substanz kann dabei dem gesamten Flüssigmedium oder im Fall der Kompartimentierung des Bioreaktors nur einem Teil des Mediums, das heißt entweder Flüssigmedium 1 oder Flüssigmedium 2, in einer geeigneten Konzentration zugegeben werden. Nach einer Substanz-spezifischen Einwirkzeit, die insbesondere zwischen drei Minuten und vier Stunden liegt, wird das Hautäquivalent mit Medium gewaschen und sein Zustand mit entsprechenden Assays erfasst, wie z. B. Lebensfähigkeitsassays. Veränderungen in der Histologie, Freisetzung von Cytokinen oder der Genregulation können ebenfalls festgehalten werden. Der parallele Ansatz von Kontrollen ist in allen Fällen unerlässlich. Ein Beispiel für einen Assay zur Bestimmung der Lebensfähigkeit von Geweben ist MTT ET-50, der auf der Reduktion einer gelben Tetrazolium-Verbindung (MTT) zu einem unlöslichem, violetten Formazon-Produkt durch die Mitochondrien lebender Zellen basiert. Die Hybridstruktur wird für mehrere Stunden mit MTT inkubiert, die Zellen lysiert und die Farbintensität, die der Zellvitalität proportional ist, bei 570 nm erfasst. ET-50 bezieht sich hierbei auf die Einwirkungszeit einer Testsubstanz, innerhalb der Lebensfähigkeit eines Gewebes um 50 % reduziert wird. Geeignete Verfahren zur Verabreichung der Testsubstanzen als auch zur Analyse ihrer Wirkungen auf Hautäquivalente sind dem Fachmann insbesondere aus standardisierten Protokollen, wie z. B. jenen der ICCVAM, bekannt.

Die Erfindung bezieht sich ferner auf die Verwendung der organotypischen Hybridstruktur für den Hautersatz. Ein weiterer Aspekt der Erfindung betrifft die Verwendung der organotypischen Hybridstruktur für Messungen des Stofftransports über eine endotheliale Barriere. Noch ein weiterer Aspekt der Erfindung betrifft die Verwendung der organotypischen Hybridstruktur für Messungen von Blutzellen/Endothel-Interaktionen. Die organotypische Hybridstruktur kann ebenfalls für Untersuchungen von Angiogeneseprozessen verwendet werden.

Es versteht sich, dass diese Erfindung nicht auf die spezifischen Methoden, Zusammensetzungen und Bedingungen beschränkt ist, wie sie hierin beschrieben sind, da solche Dinge variieren können. Es versteht sich des Weiteren, dass die vorliegend verwendete Terminologie ausschließlich dem Zweck der Beschreibung besonderer Ausführungsformen dient und nicht den Schutzumfang der Erfindung einschränken soll. Wie vorliegend in der Spezifikation einschließlich der anhängigen Ansprüche verwendet, schließen Wortformen im Singular, wie z. B. "ein", "eine", "einer", "der" oder "das" die Entsprechung im Plural ein, sofern der Kontext nicht eindeutig etwas anderes vorgibt. Beispielsweise enthält der Bezug auf "eine Hohlfasermembran" eine einzelne oder mehrere Hohlfasermembranen, die wiederum identisch oder verschieden sein können, oder der Bezug auf "ein Verfahren" schließt äquivalente Schritte und Verfahren ein, die dem Fachmann bekannt sind.

Die Erfindung wird nachfolgend in Ausführungsbeispielen anhand der zugehörigen Zeichnungen näher erläutert. Es zeigen:
- Figur 1: eine Immobilisierung von Zellen an einen Zellträger gemäß einer ersten Ausgestaltung der Erfindung;
- Figur 2: eine Immobilisierung von Zellen an einen Zellträger gemäß einer zweiten Ausgestaltung der Erfindung;
- Figur 3: einen Bioreaktor zur Erzeugung einer Hybridstruktur der Haut;
- Figur 4: verschiedene Hybridstrukturen der Haut auf einer Hohlfasermembran, und
- Figur 5: eine Messung des transepithelialen Widerstands.

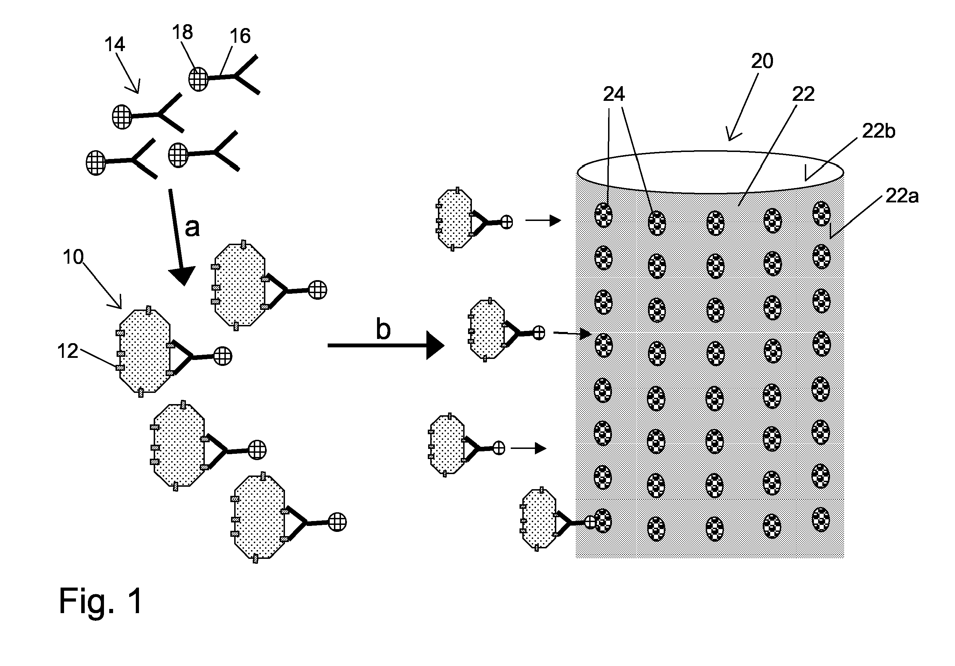

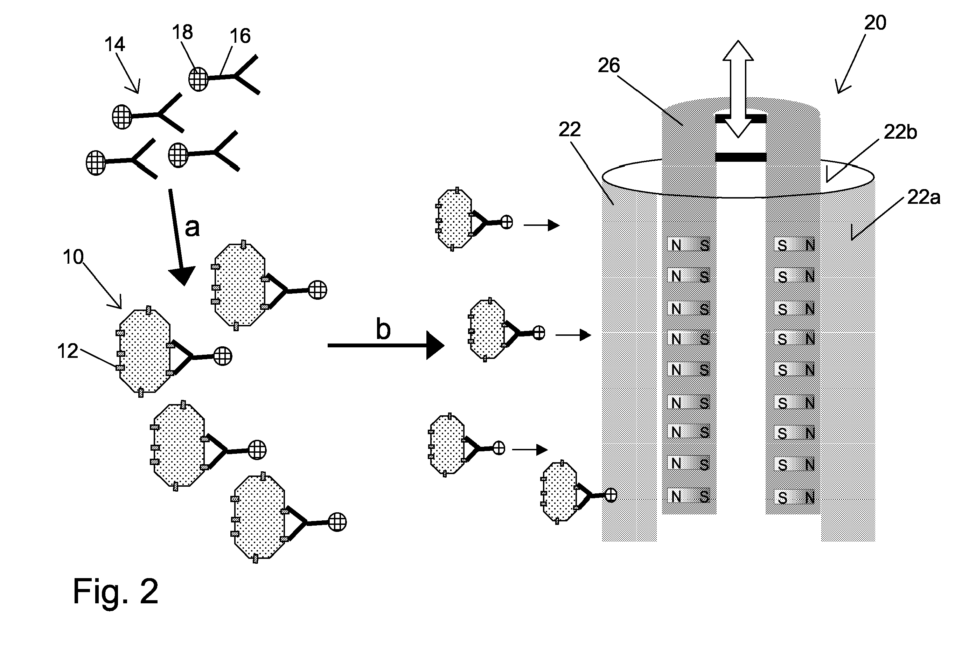

In den Figuren 1 und 2 sind mit 10 Zellen bezeichnet, die auf ihren Zelloberflächen Oberflächenproteine 12 aufweisen. Bei den Zellen 10 handelt es sich beispielsweise um Hautzellen, insbesondere um eine Einzelzellsuspension von Epithelzellen. Die Zellen 10 werden in einem ersten Schritt mit einer insgesamt mit 14 bezeichneten magnetisch-markierten Substanz in Kontakt gebracht, insbesondere in einem äquimolaren Verhältnis. Die Zellen 10 befinden sich z. B. in physiologischer Kochsalzlösung, während die magnetisch-markierte Substanz 14 in einem Phosphat-Puffer mit Magnesium-lonen und Glycerin vorliegt. Die magnetisch-markierte Substanz 14 weist einen Grundkörper 16 auf, hier ein mit dem Oberflächenprotein 12 spezifisch reagierender Antikörper 16, sowie eine magnetische Markierung 18. Beispielsweise ist der Antikörper 16 über eine Hydrazon-Bindung mit einem eine Magnetit-Füllung aufweisenden Magnetic Bead als magnetische Markierung 18 magnetisch markiert. Die Spezifität des Antikörpers 16, bei dem es sich insbesondere um einen monoklonalen Antikörper handelt, führt zu einer selektiven Bindung der Epithelzellen aus der Zellsuspension.

In einem zweiten Schritt werden die mit den Antikörpern 16 markierten Zellen 10 an einen Zellträger 20 gekoppelt. Im Beispiel der Figur 1 handelt es sich um eine Polymerhohlfaser 22 aus Polyethersulfon, in die ein magnetischer Stoff 24 eingebracht ist. Der magnetische Stoff 24 ist dabei bereichsweise in Form von Partikeln während der Herstellung der Hohlfaser 22 in selbige integriert worden. Die Partikel sind vorliegend aus Eisenoxid und weisen ein gleichmäßiges Linienmuster auf. Durch das permanente Magnetfeld der Eisenoxid-Partikel werden die Magnetic Beads angezogen, die sich an den Antikörper-markierten Epithelzellen befinden. Die Kopplung der Magnetic Beads an die magnetischen Partikel der Polymerhohlfaser 22 bewirkt die Immobilisierung der Zellen 10 in einem definierten Abstand, der durch den Partikelabstand determiniert ist. Abschließend werden sowohl nicht-markierte Zellen als auch überschüssige Antikörper 16 in Waschschritten entfernt und die immobilisierten Zellen 10 kultiviert.

Im Beispiel der Figur 2 handelt es sich um eine Polymerhohlfaser 22, in deren Hohlraum ein Ringmagnet 26 eingeführt ist. Der Ringmagnet 26 ist als Dauermagnet aus Eisen ausgeführt und ruft ein gleichmäßiges Magnetfeld hervor, das eine Anziehung auf die Magnetic Beads an den Antikörper-markierten Zellen 10 ausübt. Die Immobilisierung erfolgt vorliegend in zufälligen Abständen. Im Verlauf einer Kultivierung der Zellen 10 ist auch ein adhärentes Wachstum auf der Polymerhohlfaser 22 gegeben, so dass der Ringmagnet 26 aus der Hohlfaser entnommen werden kann. Besteht die Hohlfaser 22 beispielsweise aus Polyglycogen als resorbierbares Material kann die erzeugte epidermale Hybridstruktur als Hautersatz verwendet werden.

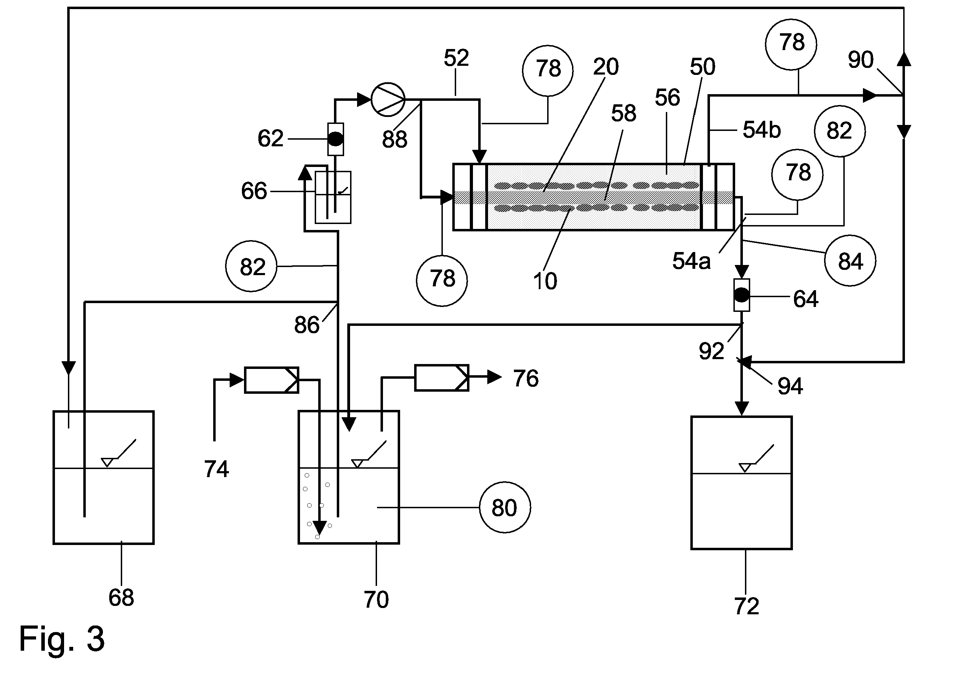

Das zentrale Element des Versuchsaufbaus in Figur 3 stellt der Bioreaktor 50 dar. Er ist mit porösen Hohlfasermembranen 20 durchzogen. Hierdurch wird eine Kompartimentierung des Bioreaktors 50 in zwei Bereiche erzielt, indem der Gewebeträger den Bioreaktor 50 in ein luminales Kompartiment (vorliegend Lumen 28 genannt), und ein basales Kompartiment (vorliegend Außenbereich 30 genannt) teilt. Sie stehen ausschließlich über die Poren der Hohlfasermembran 20 miteinander in Verbindung. Das Material der Hohlfasermembran 20 ist aus Polyethersulfon und weist eine durchschnittliche Porengröße von 0,5 µm auf. Das Lumen 28 und der Außenbereich 30 werden getrennt mit Flüssigmedium 2 (58) bzw. Flüssigmedium 1 (56) durchströmt, die vorliegend eine identische Zusammensetzung aufweisen, da sie aus der gleichen Medienvorlage 70 stammen. Als Flüssigmedium wird ein kommerzielles Basalmedium, wie z. B. M199 oder DMEM, verwendet, das mit FCS angereichert ist und außerdem Antibiotika, wie z. B. Penicillin und Streptomycin, enthält. Die Medienvorlage 70 ist mit einer Temperaturregelung TIRC 80 ausgestattet. Das in der Medienvorlage 70 befindliche Flüssigmedium wird durch Zuluft 74 mit Sauerstoff angereichert. Gleichzeitig wird die ungenutzte Abluft 76 an anderer Stelle aus der Medienvorlage 70 abgeführt. In der Animpfvorlage 68 befindet sich zumindest ein erster Zelltyp 10 in einer geeigneten Nährlösung, die hier dem Medien in der Medienvorlage 70 entspricht. Beim ersten Zelltyp 10 handelt es sich beispielsweise um Keratinozyten.

Der Bioreaktor 50 kann durch entsprechende Ventilstellung von V1 86 entweder mit Animpflösung aus der Animpfvorlage 68 oder mit Nährlösung aus der Medienvorlage 70 beaufschlagt werden. Während des Animpfvorgangs sollten die Dreiwegeventile V1 86 und V2 88 so geschaltet sein, dass ausschließlich Animpflösung aus der Animpfvorlage 68 über den Zulauf 52 in den Außenbereich 30 der Hohlfasermembran 20 gelangt. Es hat sich als zweckmäßig erwiesen, einen Teil oder auch den gesamten basalen Ablauf 54b in die Animpfvorlage 68 zurückzuführen. Hierzu sind die entsprechenden Ventilstellungen an den Dreiwegeventilen V3 90, V4 92 und V5 94 vorzunehmen. Vor der Animpfung sollte das Lumen 28 bereits mit Nährlösung aus der Medienvorlage 70 befüllt worden sein. Die Keratinozyten werden auf der außen magnetisch-funktionalisierten Oberfläche der Hohlfasermembran 20 in Anlehnung an Figur 1 immobilisiert. Die magnetisch-markierte Substanz 14 liegt hierbei zusammen mit den Zellen 10 im Medium der Animpfvorlage 68 vor. Die Immobilisierung kann sowohl im Batch-Verfahren als auch kontinuierlich erfolgen. Verbrauchtes Medium und nicht immobilisierte Zellen 10 werden als Ablauf 54 aus dem Bioreaktor 50 entfernt. Durch den Betrieb des Bioreaktors 50 im Kreislauf wird eine bessere Verstoffwechslung der einzelnen Medienbestandteile und eine dichtere Immobilisierung der Zellen 10 auf der Hohlfasermembran 20 erreicht. Nach erfolgter Immobilisierung wird die Animpfvorlage 68 abgeschaltet und der Bioreaktor 50 ausschließlich mit Medium aus der Medienvorlage 70 beschickt.

Durch entsprechende Ventilstellung von V1 86 und V2 88 wird Medium aus der Medienvorlage 70 ausschließlich in das Lumen 28 gefördert. Störende Luftblasen können mit der Blasenfalle 66 dem Medium entzogen werden. Für den weiteren Betrieb kann das unverbrauchte Nährmedium aus dem luminalem Ablauf 54a zurück in die Medienvorlage 70 gefördert werden (geschlossenes System) oder in der Abfallvorlage 72 gesammelt und anschließend verworfen werden (offenes System). Die Keratinozyten metabolisieren Bestandteile des Mediums und wachsen zu einer konfluenten Zellschicht. Am Ablauf 54 wird das Medium kontinuierlich in die Abfallvorlage 72 überführt oder wie oben beschrieben über eine entsprechende Ventilsteuerung im Kreislauf gefahren. Zur Stratifizierung der Keratinozyten kann die Animpfvorlage 68 wieder zugeschaltet werden. Die einzelnen Bereiche im Versuchsaufbau sind an geeigneten Stellen mit Möglichkeiten der Probenahme (Probenahme 1 [62] und Probenahme 2 [64]) sowie Mess- und Regeltechnik versehen. Hervorzuheben sind die online Überwachung von Sauerstoff (QIR pO₂ 82) und Kohlendioxid (QIR pCO₂ 84) in der flüssigen Phase, um zeitnahe Informationen über den metabolischen Zustand der Zellen 10 zu gewinnen. Druckverluste über die Verfahrensstrecke werden mittels PIR 78 in den beiden Kompartimenten gemessen. Hiermit lassen sich frühzeitig Aussagen hinsichtlich der Strömungsbedingungen im Bioreaktor 50 machen, die Verstopfungen lokalisieren oder die Berechnung von Scherkräften und Fließgeschwindigkeiten erlauben.

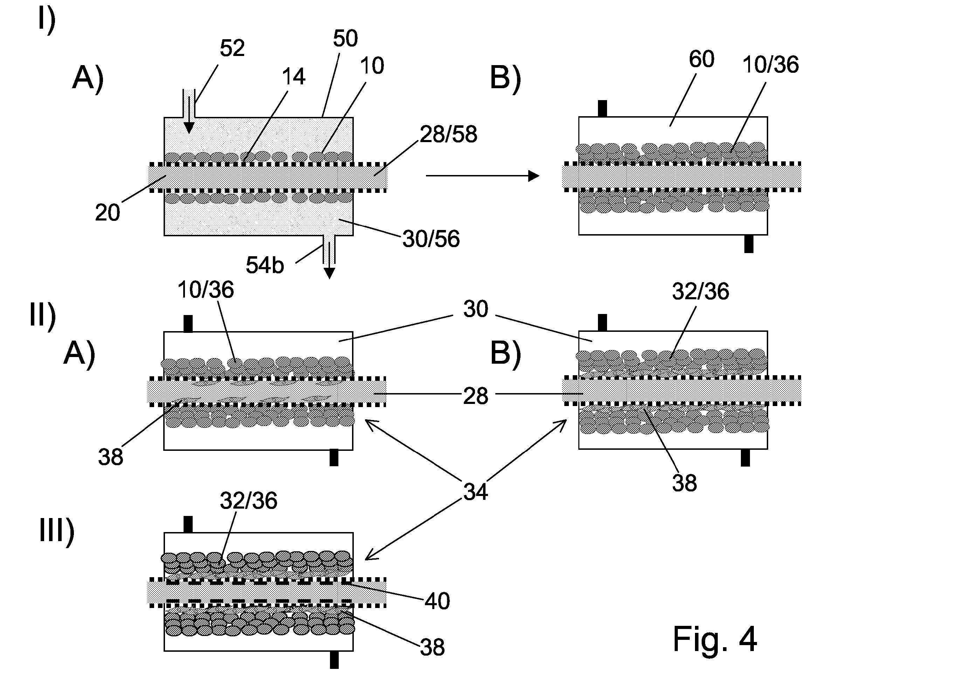

Die Hybridstrukturen 34 der Figur 4 beinhalten eine zunehmende Komplexität von der epidermalen Struktur 36 (I), über die epidermale 36 und dermale Struktur 38 (II) bis hin zur epidermalen 36, dermalen 38 und endothelialen Struktur 40 (III). Keratinozyten werden zunächst in Anlehnung an Figur 1 auf der äußeren Oberfläche 23a der Hohlfasermembran 20 magnetisch immobilisiert und in Anlehnung an Figur 3 zu einer konfluenten Zellschicht kultiviert (IA). Anschließend wird das Flüssigmedium 1 (56) komplett aus dem Bereich zwischen der Innenwand des Bioreaktors 50 und der Außenwand 23a der Hohlfasermembran 20 abgelassen und durch eine Gasphase 60 ersetzt. Im Zuge der kontrollierten Begasung mit einer definierten Sauerstoff/Kohlendioxid-Mischung wird die Stratifizierung der Keratinozyten initiiert, so dass eine organotypische epidermale Struktur 36 aus mehreren Schichten erzeugt wird (IB). Im folgenden Schritt wird das Lumen 28 der Hohlfasermembran 20 mit Fibroblasten als zweitem Zelltyp 32 in einem Flüssigmedium 2 (58) beimpft. Die Fibroblasten wachsen adhärent auf der innen liegenden Oberfläche 23b der Hohlfasermembran 20 zu einer dermalen Struktur 38 (IIA). Alternativ werden die epidermale 36 und dermale Struktur 38 nacheinander im selben Kompartiment des Bioreaktors 50 generiert, so dass insbesondere deren Ablösung von der Hohlfasermembran 20 und Verwendung als Hautersatz vereinfacht ist. Dazu werden zunächst Fibroblasten entsprechend Figur 4 IA immobilisiert und konfluent kultiviert. Im Anschluss werden Keratinozyten auf der erhaltenen dermalen Struktur 38 durch adhärentes Wachstum angesiedelt und analog Figur 4 IB zur epidermalen Struktur 36 stratifiziert (IIB). Ausgehend von letztgenannter Hybridstruktur 34 der Figur 4 IIB wird danach das Anwachsen von Endothelzellen auf der innen liegenden Oberfläche 23b der Hohlfasermembran 20 analog zu Figur 4 IIA induziert, um beispielsweise Angiogeneseprozesse zu simulieren (III).

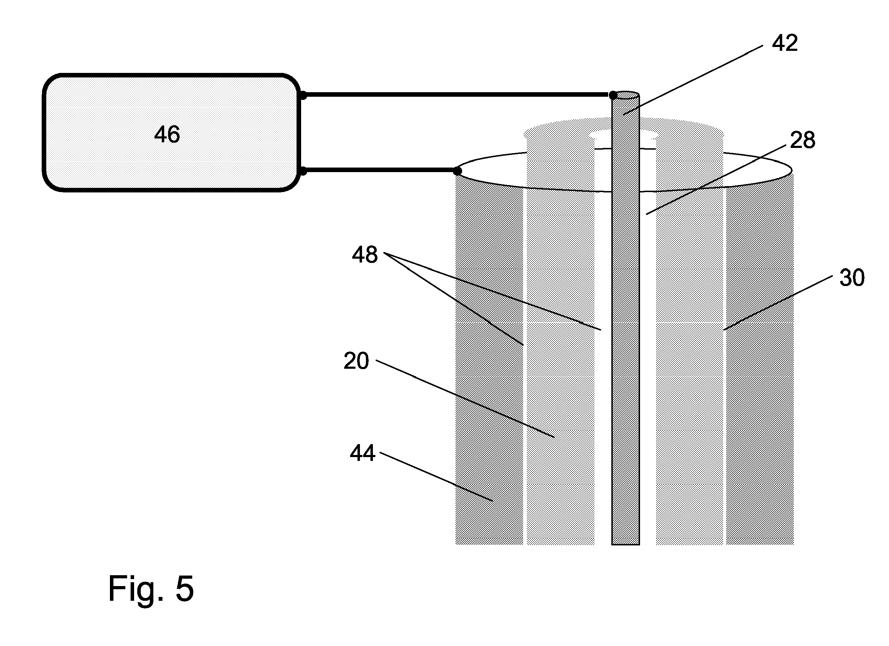

Zur Messung des transzellulären Widerstands, wie sie in Figur 5 dargestellt ist, wird eine Messzelle axialsymmetrisch ähnlich dem Bioreaktor 50 (siehe Figur 3) angeordnet. Die Innenwand der Messzelle wird als Ringblechelektrode 44 aus Platin ausgeführt. Als Gegenelektrode 42 wird ein Draht, der hier ebenfalls aus Platin besteht, in das Lumen 28 der Hohlfasermembran 20 eingeführt. Bei der Hohlfasermembran 20 handelt es sich im Beispiel um eine Polymerhohlfaser 22 aus Polyethersulfon. Beide Elektroden sind über einen Stromkreis mit einer nicht dargestellten Spannungsquelle, die hier auf Wechselstrom basiert, und mit einem Widerstandsmessgerät 46, das ebenfalls mit Wechselstrom arbeitet, verbunden. Die Räume zwischen der Ringblechelektrode 44 und der Außenwand 23a der Hohlfasermembran 20, das heißt der Außenbereich 30, sowie zwischen der Gegenelektrode 42 und der Innenwand 23b der Hohlfasermembran 20, das heißt das Lumen 28, werden mit Elektrolytlösung 48 durchströmt. Entsprechend dem erfindungsgemäßen Verfahren wird eine Reinkultur von Keratinozyten immobilisiert, kultiviert und stratifiziert. Durch Vergleich der Widerstandsmesswerte der juvenilen Hohlfasermembran 20 mit jenen Werten der mit Zellen besiedelten Hohlfasermembran 20, die sich auch in einer aufgenommenen zellspezifischen Eichkurve wiederfinden, wird auf die Anzahl der Polyschichten und die Dicke der Hybridstruktur 34 geschlossen. Die Stratifizierung wird bis zur gewünschten epidermalen Struktur 36 durchgeführt und dann gestoppt.

### BEZUGSZEICHENLISTE

- 10: Zelltyp / Zelle
- 12: Oberflächenprotein
- 14: magnetisch-markierte Substanz
- 16: Grundkörper / Antikörper
- 18: magnetischer Körper
- 20: Hohlfasermembran / Zellträger
- 22: Polymerhohlfaser
- 23a: außen liegende Oberfläche Hohlfasermembran
- 23b: innen liegende Oberfläche Hohlfasermembran
- 24: magnetischer Stoff
- 26: Ringmagnet
- 28: Lumen Hohlfasermembran
- 30: Außenbereich Hohlfasermembran
- 32: zweiter Zelltyp
- 34: Hybridstruktur
- 36: epidermale Struktur
- 38: dermale Struktur
- 40: endotheliale Struktur
- 42: Gegenelektrode
- 44: Ringblechelektrode
- 46: Widerstandsmessgerät
- 48: Elektrolytlösung
- 50: Bioreaktor
- 52: Zulauf
- 54a: luminaler Ablauf
- 54b: basaler Ablauf
- 56: Flüssigmedium 1
- 58: Flüssigmedium 2
- 60: Gasphase
- 62: Probenahme 1
- 64: Probenahme 2
- 66: Blasenfalle
- 68: Animpfvorlage
- 70: Medienvorlage
- 72: Abfallvorlage
- 74: Zuluft
- 76: Abluft
- 78: PIR (Pressure Indicate Register)
- 80: TIRC (Temperature Indicate Register Control)
- 82: QIR (Analysewert Indicate Register) pO₂
- 84: QIR (Analysewert Indicate Register) pCO₂
- 86: Dreiwegeventil V1
- 88: Dreiwegeventil V2
- 90: Dreiwegeventil V3
- 92: Dreiwegeventil V4
- 94: Dreiwegeventil V5

## Patentansprüche

1. Verfahren zur Erzeugung einer organotypischen Hybridstruktur (34) der Haut mit den Schritten:
(a) Immobilisierung von zumindest einem ersten Zelltyp (10) auf einer Hohlfasermembran (20), und
(b) Kultivierung des zumindest einen ersten Zelltyps (10) in dynamischer Kultur über eine erste Kultivierungsdauer zu einer mehrschichtigen organotypischen Gewebestruktur der Haut.

2. Verfahren nach Anspruch 1,
**dadurch gekennzeichnet, dass**
sich nach Schritt (b) weitere Schritte anschließen:
(c) Immobilisierung von zumindest einem zweiten Zelltyp (32) auf der Hybridstruktur (34) und/oder auf der Hohlfasermembran (20),
(d) Kultivierung des zumindest einen zweiten Zelltyps (32) in dynamischer Kultur über eine zweite Kultivierungsdauer, und optional
(e) Wiederholung der Schritte (c) und (d) mit zumindest einem weiteren Zelltyp.

3. Verfahren nach einem der vorhergehenden Ansprüche,
**dadurch gekennzeichnet, dass**
sich nach Schritt (b), (d) und/oder (e) ein weiterer Schritt anschließt:
(f) Kultivierung der Hybridstruktur (34) in dynamischer Kultur über eine dritte Kultivierungsdauer.

4. Verfahren nach einem der vorhergehenden Ansprüche,
**dadurch gekennzeichnet, dass**
als der zumindest eine erste Zelltyp (10), als der zumindest eine zweite Zelltyp (32) und/oder als der zumindest eine weitere Zelltyp eine adulte Stammzelle, Progenitorzelle und/oder ein ausdifferenzierter Hautzelltyp immobilisiert werden, vorzugsweise basale Keratinozyten, Keratinozyten und/oder Fibroblasten, besonders bevorzugt basale Keratinozyten und/oder Keratinozyten.

5. Verfahren nach Anspruch 4,
**dadurch gekennzeichnet, dass**
die adulte Stammzellen und/oder Progenitorzellen nach Schritt (a) oder (c) ausdifferenziert werden.

6. Verfahren nach einem der vorhergehenden Ansprüche,
**dadurch gekennzeichnet, dass**
die basalen Keratinozyten und/oder Keratinozyten in dynamischer Kultur über eine vierte Kultivierungsdauer stratifiziert werden.

7. Verfahren nach einem der vorhergehenden Ansprüche,
**dadurch gekennzeichnet, dass**
als Hybridstruktur (34) eine epidermale Struktur (36), eine dermale Struktur (38), oder eine epidermale (36) und dermale Struktur (38) erzeugt werden, vorzugsweise eine epidermale (36) und eine zwischen Hohlfasermembran (20) und epidermaler Struktur (36) angeordnete dermale Struktur (38).

8. Verfahren nach einem der vorhergehenden Ansprüche,
**dadurch gekennzeichnet, dass**
in Schritt (a) zumindest zwei erste Zelltypen (10) und/oder in Schritt (c) zumindest zwei zweite Zelltypen (32) co-immobilisiert werden.

9. Verfahren nach Anspruch 8,
**dadurch gekennzeichnet, dass**
als einer der zumindest zwei ersten Zelltypen (10) und/oder einer der zumindest zwei zweiten Zelltypen (32) Endothelzellen immobilisiert werden.

10. Verfahren nach einem der vorhergehenden Ansprüche,
**dadurch gekennzeichnet, dass**
die Hohlfasermembran (20) auf ihrer außen liegenden Oberfläche (23a) mit einer endothelialen Struktur (40) beschichtet wird.

11. Verfahren nach Anspruch 9 oder 10,
**dadurch gekennzeichnet, dass**
als Hybridstruktur (34) eine epidermale (36) und endotheliale Struktur (40); eine dermale (38) und endotheliale Struktur (40); oder eine epidermale (36), dermale (38) und endotheliale Struktur (40) erzeugt werden; vorzugsweise eine epidermale (36) und endotheliale Struktur (40); oder eine epidermale (36), dermale (38) und endotheliale Struktur (40).

12. Verfahren nach einem der vorhergehenden Ansprüche,
**dadurch gekennzeichnet, dass**
als Hohlfasermembran (20) eine Polymerhohlfaser (22) verwendet wird, vorzugsweise aus Polysulfon, Polyethersulfon, Polysulfonpolyvinylpyrollidinon, Polyamid, Acrylnitril, Polyacrylnitril, Polyetherimid, Polylactid, Polyglykolid, Polypeptid oder Derivaten davon, besonders bevorzugt aus Polyethersulfon, Polyetherimid, Polylactid oder Polyglycogen.

13. Verfahren nach einem der vorhergehenden Ansprüche,
**dadurch gekennzeichnet, dass**
eine Hohlfasermembran (20) mit einer Porengröße von höchstens 0,5 µm verwendet wird, vorzugsweise höchstens 0,1 µm.

14. Verfahren nach einem der vorhergehenden Ansprüche,
**dadurch gekennzeichnet, dass**
der zumindest eine erste Zelltyp (10) ausschließlich auf der außen liegenden Oberfläche (23a) der Hohlfasermembran (20) immobilisiert und kultiviert wird.

15. Verfahren nach einem der vorhergehenden Ansprüche,
**dadurch gekennzeichnet, dass**
der zumindest eine erste Zelltyp (10) magnetisch immobilisiert wird, wobei gleichzeitig oder in beliebiger Reihenfolge an den ersten Zelltyp (10) zumindest eine magnetisch-markierte Substanz (14) gekoppelt wird, und die zumindest eine magnetisch-markierte Substanz (14) an eine zumindest bereichsweise magnetische Hohlfasermembran (20) gekoppelt wird.

16. Verfahren nach einem der vorhergehenden Ansprüche,
**dadurch gekennzeichnet, dass**
die Dichte der Hybridstruktur (34) durch die Messung des transzellulären Widerstands über die Hohlfasermembran (20) in einer Messzelle kontrolliert wird, wobei die Messzelle aus einer Gegenelektrode (42) im Lumen (28) der Hohlfasermembran (20) und einer Ringblechelektrode (44) im Außenbereich (30) der Hohlfasermembran (20), die beide mit einem Widerstandsmessgerät (46) verbunden sind, und Elektrolytlösung (48) im Lumen (28) und Außenbereich (30) besteht.

17. Verfahren nach einem der vorhergehenden Ansprüche,
**dadurch gekennzeichnet, dass**
die dynamische Kultur im Kreislauf und/oder als Gradientenkultur durchgeführt wird.

18. Organotypische Hybridstruktur (34), hergestellt nach einem der Ansprüche 1 bis 17.

19. Organotypische Hybridstruktur (34), umfassend
(a) eine Hohlfasermembran (20) mit einer innen liegenden Oberfläche (23b) und einer außen liegenden Oberfläche (23a), und
(b) eine zumindest auf der außen liegenden Oberfläche (23a) angeordnete mehrschichtige Gewebestruktur aus zumindest einem ersten Zelltyp (10).

20. Organotypische Hybridstruktur (34) nach Anspruch 19,
**dadurch gekennzeichnet, dass**
die Gewebestruktur eine epidermale Struktur (36) aus Keratinozyten als ersten Zelltyp (10) aufweist.

21. Organotypische Hybridstruktur (34) nach Anspruch 19 oder 20,
**dadurch gekennzeichnet, dass**
die Gewebestruktur zumindest einen zweiten Zelltyp (32) und optional zumindest einen weiteren Zelltyp enthält.

22. Organotypische Hybridstruktur (34) nach Anspruch 20 und 21,
**dadurch gekennzeichnet, dass**
die Gewebestruktur eine dermale Struktur (38) aus Fibroblasten als zweiten Zelltyp (32) aufweist, wobei die dermale Struktur (38) zwischen der außen liegenden Oberfläche (23a) und der epidermalen Struktur (36) angeordnet ist.

23. Organotypische Hybridstruktur (34) nach Anspruch 22,
**dadurch gekennzeichnet, dass**
die Gewebestruktur eine endotheliale Struktur (40) aus Endothelzellen als weiteren Zelltyp aufweist, wobei die endotheliale Struktur (40) auf der innen liegenden Oberfläche (23b) angeordnet ist.

24. Verwendung der organotypischen Hybridstruktur (34) nach einem der Ansprüche 18 bis 23 für den Hautersatz.

25. Verwendung der organotypischen Hybridstruktur (34) nach einem der Ansprüche 18 bis 23 für in-vitro Toxizitätstests.

26. Verwendung der organotypischen Hybridstruktur (34) nach einem der Ansprüche 18 bis 23 für Messungen des Stofftransports über eine endotheliale Barriere.

27. Verwendung der organotypischen Hybridstruktur (34) nach einem der Ansprüche 18 bis 23 für Messungen von Blutzellen/Endothel-Interaktionen.

28. Verwendung der organotypischen Hybridstruktur (34) nach einem der Ansprüche 18 bis 23 für Untersuchungen von Angiogeneseprozessen.
